# EUROPEAN PATENT SPECIFICATION

(11) **EP 3 934 824 B1**
(45) Date of publication and mention of the grant of the patent: **09.12.2026**
(21) Application number: 20766874.0
(22) Date of filing: 05.03.2020
(51) Int. Cl.: B21D 7/03, A61B 17/88, B29C 53/08, B29C 53/84

(54) **METHODS FOR BENDING OF ELONGATED ELEMENTS CONTAINING THERMOPLASTIC POLYMERS**
VERFAHREN ZUM BIEGEN VON LÄNGLICHEN ELEMENTEN, DIE THERMOPLASTISCHE POLYMERE ENTHALTEN
PROCÉDÉS DE CINTRAGE D'ÉLÉMENTS ALLONGÉS CONTENANT DES POLYMÈRES THERMOPLASTIQUES

(30) Priority: 07.03.2019 US 201962814873 P
(43) Date of publication of application: 12.01.2022
(73) Proprietor: CarboFix Spine Inc., Ocean Isle Beach, NC 28469 (US)
(72) Inventor: BEYAR, Mordechay, 6311901 Tel Aviv (IL); UMANSKY, Daniel, 4462707 Kfar Saba (IL); GLOBERMAN, Oren, 4691000 Kfar-Shmaryahu (IL)
(74) Representative: Pearl Cohen UK
(86) International application number: PCT/IL2020/050261
(87) International publication number: WO 2020/178838

(56) References cited:
- JP-A- 2006 150 934
- US-A- 5 405 073
- US-A- 5 938 662
- US-A1- 2004 267 260
- US-A1- 2004 267 260
- US-A1- 2009 326 582
- US-A1- 2010 101 295
- US-A1- 2010 101 295
- US-A1- 2010 318 130
- US-A1- 2011 265 538
- US-A1- 2011 270 262
- US-A1- 2016 001 039
- US-A1- 2017 239 877
- US-A1- 2018 117 290

## Description

### FIELD OF THE INVENTION

The present invention relates to the field of methods of bending elongated assemblies and, more particularly, to methods of bending elongated assemblies containing thermoplastic polymers.

### BACKGROUND OF THE INVENTION

An elongated element containing thermoplastic polymers must be heated in order to be shaped. Shaping of the heated elongated element may undesirably distort a cross-sectional profile of the elongated element in a shaping region.

US 2010/318130 discloses a flexible spinal fixation rod assembly that provides relatively stiff bending compliance provided by a core rod or tube that is connected to surrounding spring and associated couplings in such a way that when the core rod is bent, the spring extends and the associated couplings move away from each other, with the core rod generating a force tending to return the core rod to its unbent configuration thereof and the spring generating a force tending to return the couplings to there unextended configuration thereof.

US 2010/101295 discloses systems and methods for bending an elongate member comprising: an insulated fabric suitably sized to cover a select portion of the elongate surgical member; and a heating element to apply heat to the select portion of the elongate surgical member.

JP 2006150934 discloses a pipe bending apparatus that is used to bend a resin pipe into a predetermined shape, and is formed in a long shape so that it can be inserted into a resin pipe and fitted inside, and is rigid and long in the lateral direction. The pipe bending apparatus comprises a pipe inner shape retaining heating member configured to be bendable in a vertical direction.

### SUMMARY OF THE INVENTION

The invention is set out in the appended set of claims.

These, additional, and/or other aspects and/or advantages of the present invention are set forth in the detailed description which follows, possibly inferable from the detailed description, and/or learnable by practice of the present invention.

### BRIEF DESCRIPTION OF THE DRAWINGS

For a better understanding of embodiments of the invention and to show how the same can be carried into effect, reference will now be made, purely by way of example, to the accompanying drawings in which like numerals designate corresponding elements or sections throughout.

In the accompanying drawings:
**Figs. 1A****,** **1B****,** **1C, 1D** and **1E** are schematic illustrations of a device for maintaining a shape of a transverse cross-sectional profile of an elongated element that are useful for understanding the invention;
**Figs. 2A, 2B, 2C****,** **2D, 2E** and **2F** are schematic illustrations of various configurations of members of a device for maintaining a shape of a transverse cross-sectional profile of an elongated element that are useful for understanding the invention;
**Figs. 3A** and **3B** are schematic illustrations of a coiled device for maintaining a shape of a transverse cross-sectional profile of an elongated element that are useful for understanding the invention;
**Figs. 3C** and **3D** are schematic illustrations of an assembly including two coiled devices for maintaining a shape of a transverse cross-sectional profile of an elongated element that are useful for understanding the invention;
**Figs. 4A****,** **4B** and **4C** are schematic illustrations of an assembly including a device for maintaining a shape of a transverse cross-sectional profile of an elongated element and an envelope that are useful for understanding the invention;
**Fig. 5** is a schematic illustration of a heating device for heating an elongated assembly that is useful for understanding the invention;
**Figs. 6A****,** **6B** and **6C** are schematic illustrations of a bending device for bending an elongated assembly that is useful for understanding the invention;
**Figs. 7A****,** **7B****,** **7C****,** **7D** and **7E** are schematic illustrations of a bending device for bending an elongated assembly and including bending units with mechanically adjustable driving assemblies that are useful for understanding the invention;
**Figs. 8A** which is a schematic illustration of a bending device for bending an elongated assembly with bending units having motorized driving assemblies that is useful for understanding the invention;
**Figs. 8B****,** **8C****,** **8D** and **8E** are schematic illustrations of a single motorized bending unit of a bending device for bending an elongated assembly that are useful for understanding the invention;
**Figs. 9A** and **9B** are schematic illustrations of a bending device capable of providing three-dimensional bending of an elongated assembly that are useful for understanding the invention;
**Fig. 10A** is a block diagram of a system for bending an elongated assembly that is useful for understanding the invention;
**Figs. 10B** and **10C** are schematic illustrations of various configurations of coupling of a heating device and a bending device that are useful for understanding the invention;
**Fig. 11** is a schematic illustration of a device for determining a desired bending profile of an elongated element that is useful for understanding the invention; and
**Fig. 12** is a flowchart of a method of bending of an elongated assembly, according to some embodiments of the invention.

It will be appreciated that, for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE INVENTION

In the following description, various aspects of the present invention are described. For purposes of explanation, specific configurations and details are set forth in order to provide a thorough understanding of the present invention.

Unless specifically stated otherwise, as apparent from the following discussions, it is appreciated that, throughout the specification, discussions utilizing terms such as "processing", "computing", "calculating", "determining", "enhancing" or the like, refer to the action and/or processes of a computer or computing system, or similar electronic computing device, that manipulates and/or transforms data represented as physical, such as electronic, quantities within the computing system's registers and/or memories into other data similarly represented as physical quantities within the computing system's memories, registers or other such information storage, transmission or display devices. Any of the disclosed modules or units can be at least partially implemented by a computer processor.

Generally, devices, systems, kits and methods for shaping of an elongated element are disclosed.

The present invention provides a method of bending of an elongated assembly, the method comprising: providing an elongated assembly comprising: an elongated orthopedic implant comprising a thermoplastic polymer, the elongated orthopedic implant comprising a bending region in which the elongated orthopedic implant is to be bent, the bending region having a length; and a transverse cross-sectional profile maintaining device surrounding completely and tightly supporting the elongated orthopedic implant along the entire length of the bending region of the elongated orthopedic implant for maintaining a shape and preventing distortion of a transverse cross-sectional profile of the elongated orthopedic implant in the bending region upon heating; heating the elongated assembly using a heating device to provide a heated elongated assembly; configuring a bending device according to a desired bending profile of the elongated orthopedic implant; bending the heated elongated assembly by the bending device to provide a bended elongated assembly comprising a bended elongated orthopedic implant having the desired bending profile while maintaining the shape and preventing the distortion of the transverse cross-sectional profile of the elongated orthopedic implant in the bending region by the transverse cross-sectional profile maintaining device; cooling the bended elongated assembly; and releasing the device for maintaining the shape of the transverse cross-sectional profile from the elongated orthopedic implant.

The term "elongated element" as used herein below relates to any elongated object that contains one or more thermoplastic polymers. For example, the elongated element may contain a polyether ether ketone (PEEK) polymer. In another example, the elongated element may contain about 60% carbon and about 40% PEEK polymer.

For example, the elongated orthopedic implant may be any one from a list including an orthopedic rod (e.g., for a pedicle screw assembly), orthopedic plate (e.g., femur plate, humerus plate, etc.), K-Wire, orthopedic nails (e.g., humerus nail, cephalomedullary nail, etc.), etc.

The elongated orthopedic implant containing thermoplastic polymer(s) should be heated or pre-heated in order to be shaped. Shaping of the elongated orthopedic implant may be any mechanical action intended to change an initial shape of the elongated orthopedic implant. For example, the shaping may include any or a combination of bending, twisting, stretching and/or compression of the elongated orthopedic implant. The description below provides bending as an example for shaping. It is noted that the devices, systems, kits and methods described below may be also utilized for other types or combination of shaping (e.g., such as twisting, stretching and/or compression).

Shaping of heated elongated orthopedic implant may, for example, distort a cross-sectional profile of elongated orthopedic implant, for example in a shaping region. For example, when heated elongated orthopedic implant having an initial circular cross-section (e.g., rod) is being bent, the cross-sectional profile of the elongated element in a bending region may be distorted and may change from circular to, for example, oval or elliptic.

The disclosed devices, systems, kits and methods may enable shaping of the elongated elements containing thermoplastic polymers while preventing distortion of the shape of the cross-sectional profile in the shaping region. Furthermore, the disclosed devices and systems may be located in the operation room and may enable shaping of the elongated elements thereof in the operation room during the operational procedure.

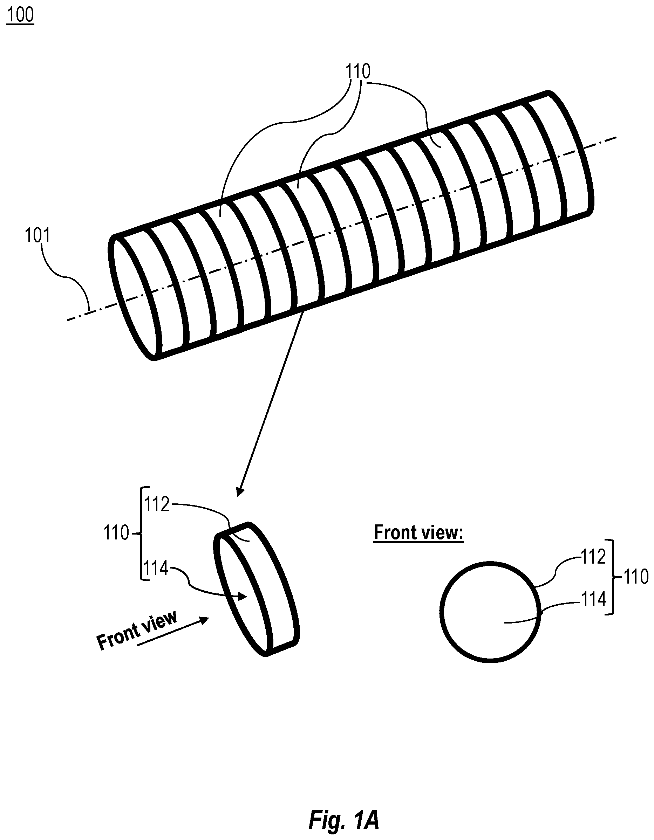

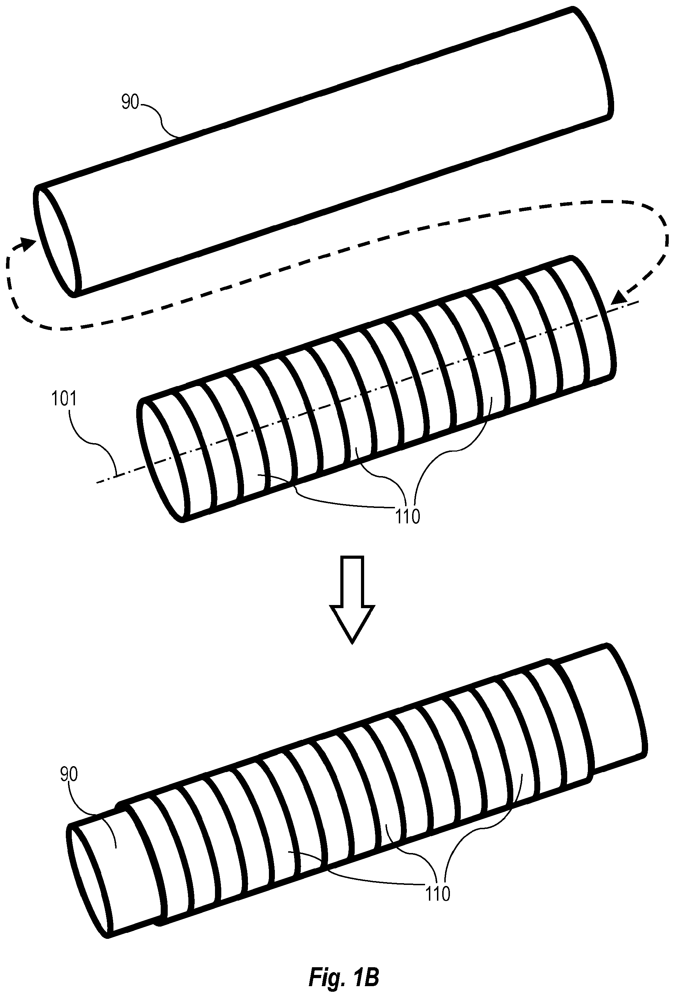

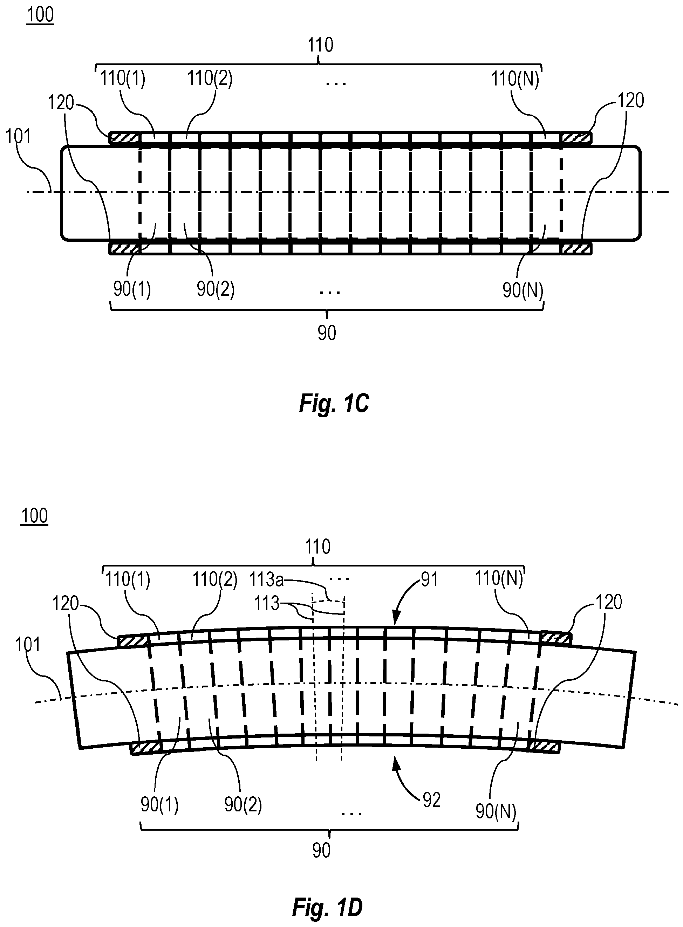

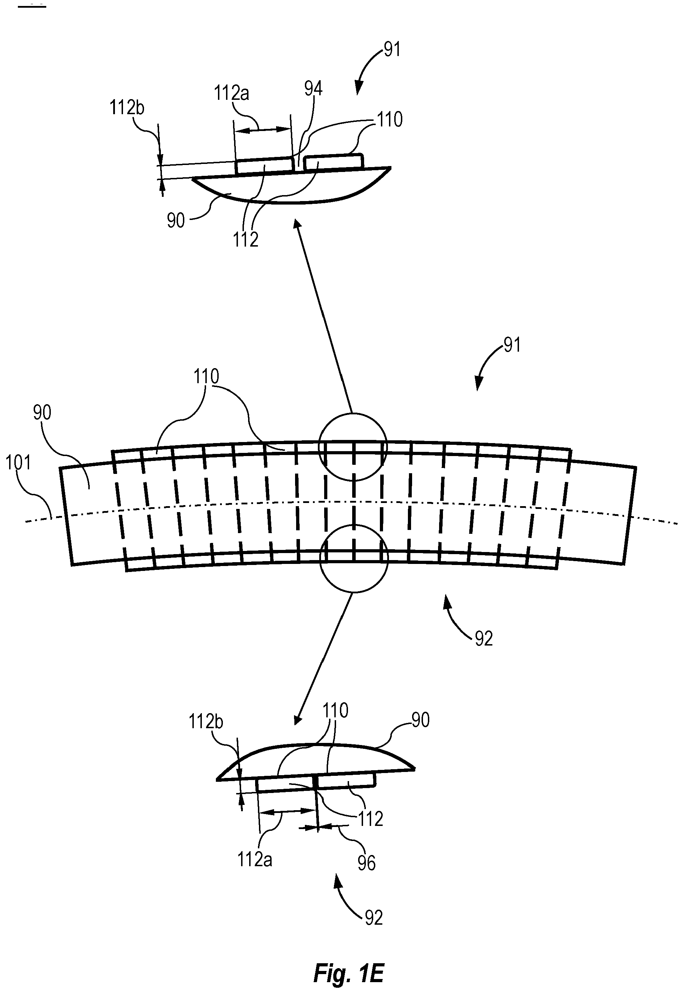

Reference is now made to **Figs. 1A****,** **1B****,** **1C, 1D** and **1E**, which are schematic illustrations of a device 100 for maintaining a shape of a transverse cross-sectional profile of an elongated element 90 useful for understanding the invention.

Device 100 may include multiple members 110 (e.g., as shown in Figs. 1A, 1B, 1C, 1D and 1E). Members 110 may be arranged along a longitudinal axis 101 of device 100. Members 110 may be adjacent to each other (e.g., may touch each other). Members 110 may be arranged along longitudinal axis 101 in a way that enables a desired measure of relative motion (e.g., lateral motion, longitudinal motion and/or angled motion) of members 110 with respect to each other.

Each of multiple members 110 may include a peripheral member portion 112 that may at least partly surround a central member opening 114 of respective member 110 (e.g., as shown in Fig. 1A).

Members 110 may be threaded onto elongated element 90 or elongated element 90 may be inserted through central member openings 114 of members 110 such that each of members 110 surrounds and tightly supports a corresponding portion of elongated element 90 (e.g., as indicated by dashed arrow in Fig. 1B).

For example, a first member 110(1) may receive and tightly support a first portion 90(1) of elongated element 90, a second member 110(2) may receive and tightly support a second portion 90(2) of elongated element 90, and/or an N-th member 110(N) may receive and tightly support an N-th portion 90(N) of elongated element 90 (e.g., as shown in Fig. 1C).

Shape and dimensions of members 110 and/or of peripheral member portions 112 and/or of central member openings 114 of members 110 may mate with the shape and dimensions of, for example, cross-sectional profile of the corresponding portions of elongated element 90 to provide the tight receipt and support of elongated element 90 therein.

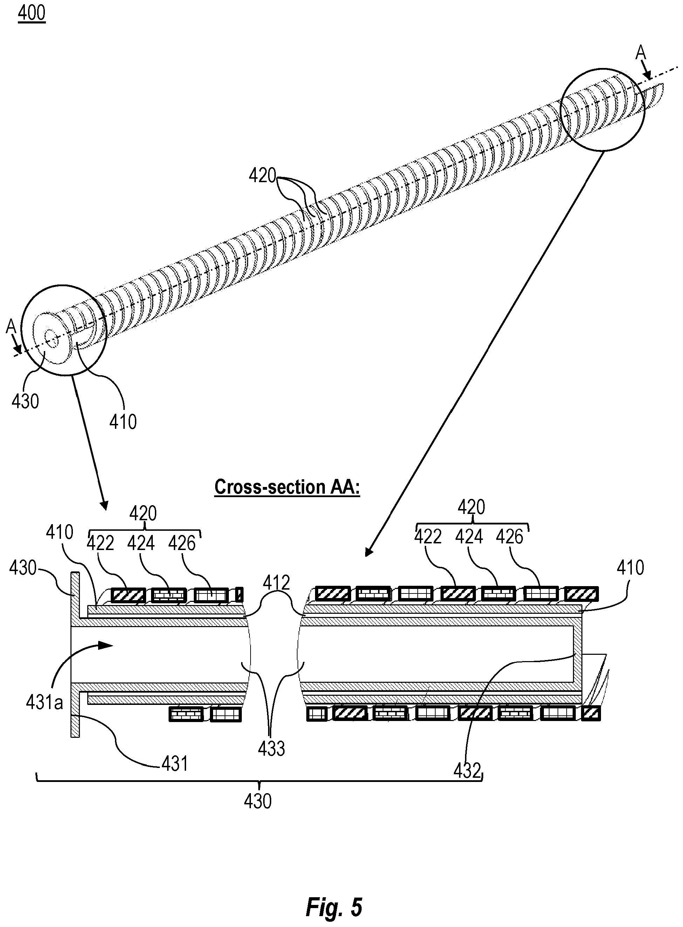

Elongated element 90 supported within device 100 may be heated (or pre-heated) such that a temperature of elongated element 90 is elevated up to a predetermined temperature value (e.g., as described below with respect to Fig. 5). The predetermined temperature value may be 380°C. Members 110 of device 100 may be made of a material that does not undergo thermal deformation at the predetermined temperature value thereof. Members 110 may be made of biocompatible material. For example, members 110 may be made of stainless steel or titanium.

Device 100 may enable bending elongated element 90 while preventing distortion/change of the cross-sectional profile of elongated element 90 in the bending region.

When bending forces are applied to elongated element 90 supported within device 100, members 110 may move with respect to each other, e.g., by getting farther or closer to each other or by changing the relative angle 113a between planes 113 of two adjacent members, while remaining unmovable (or substantially unmovable) with respect to the corresponding portions of elongated element 90 (e.g., due to tight support thereof, as described above), thereby allowing bending of elongated element 90 (e.g., as shown in Fig. 1D). Members 110 do not deform (or substantially do not deform) during bending (as members 110 may move with respect to each other and do not undergo thermal deformation), thereby preventing distortion of the cross-sectional profile of the corresponding portion of elongated element 90 and thereby maintaining (or substantially maintaining) the shape of the cross-sectional profile of elongated element 90 along the entire bending region.

Device 100 may include one or more stoppers 120 (e.g., as shown in Figs. 1C and 1D). Stoppers 120 may, for example, affix first member 110(1) and N-th member 110(N) at both ends of device 100 to, for example, further secure first member 110(1) and N-the member 110(N) in their respective positions and prevent sliding of the members thereof, for example, during bending of elongated element 90.

Upon bending of elongated device 90 supported within device 100, gaps 94 may be formed between adjacent members 110 on a convex side 91 of bended elongated element 90, while on a concave side 92 thereof members 110 may remain adjacent (or substantially adjacent) with respect to each other (e.g., as shown in Fig. 1E). For example, gaps 96 on concave side 92 may be smaller than gaps 94 on convex side 91 of elongated element 90 by at least one order of magnitude.

Members 110 may be shaped to ensure that gaps 94 between adjacent members 110 formed on convex side 91 of bended elongated element 90 do not exceed a predetermined gap threshold value.

Members 110 / peripheral portions 112 of members 110 may be made of a metal wire (e.g., stainless steel or titanium) having a rectangular (or substantially rectangular) cross-section (e.g., as shown in Figs. 1C, 1D and 1E). The rectangular cross-section of peripheral portions 112 of members 110 may have a first dimension 112a (e.g., in a direction along, or substantially along, longitudinal axis 101 of device 100) and a second dimension 112b that is perpendicular to first dimension 112a (e.g., as shown in Fig. 1E).

First dimension 112a of the cross-section of peripheral portion 112 of members 110 may range between 0.1-3.0 mm. Second dimension 112b of the cross-section of peripheral portion 112 of members 110 may range between 0.05-0.3 mm.

The predetermined gap threshold value may be no more than 0.05 mm. The predetermined gap threshold value may be no more than 0.5 mm.

When the bending of elongated element 90 is complete, device 100 may be released (e.g., slidably removed) from elongated element 90.

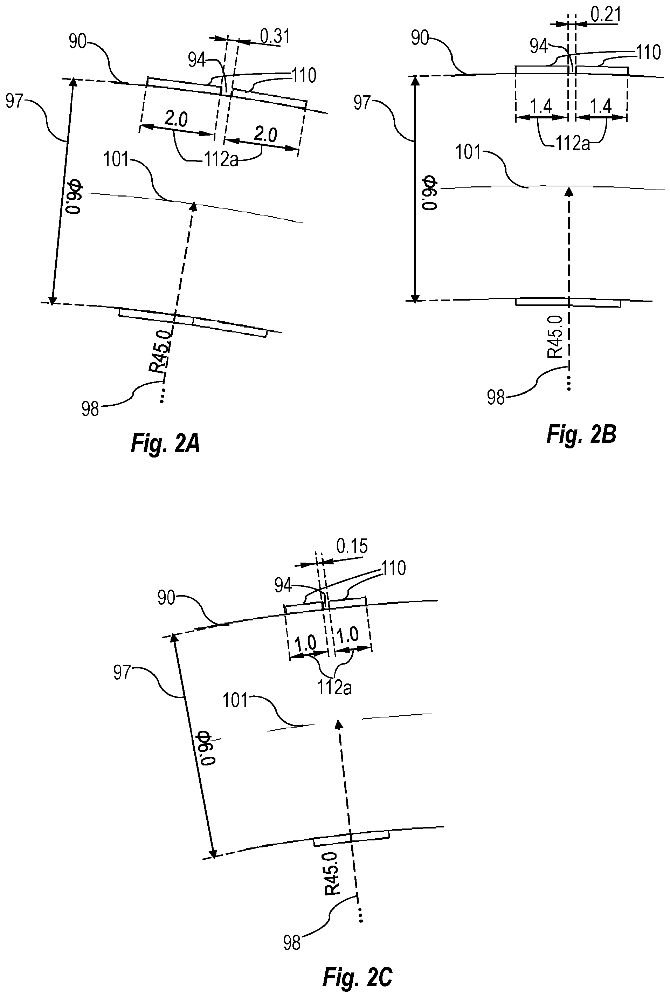

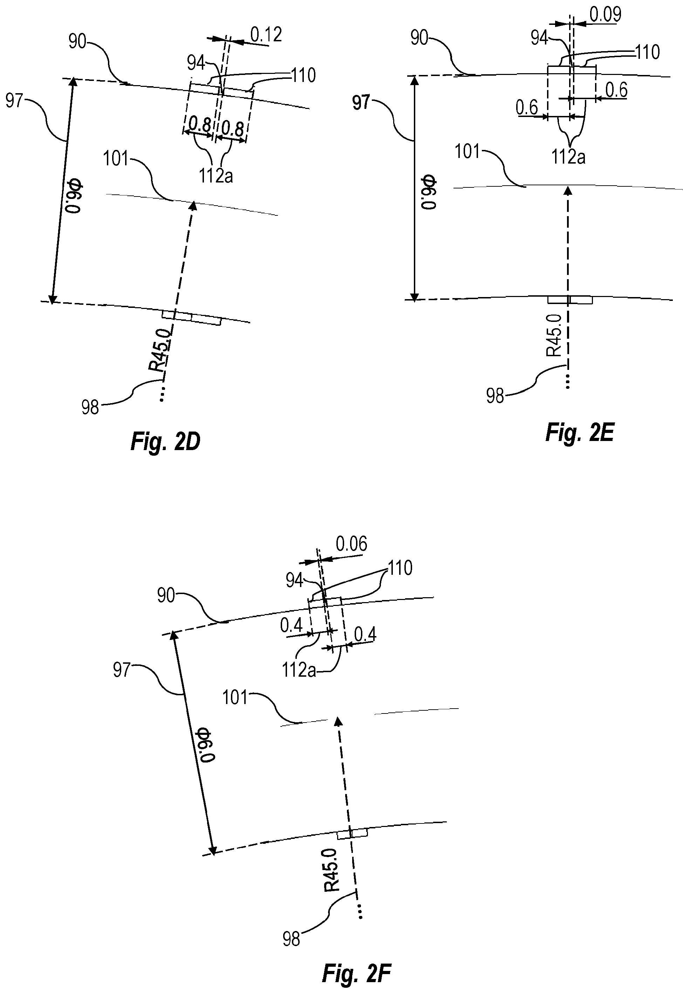

Reference is now made to Figs. 2A, 2B, 2C, 2D, 2E and 2F, which are schematic illustrations of various configurations of members 110 of a device 100 for maintaining a shape of a transverse cross-sectional profile of an elongated element 90, useful for understanding the invention.

The smaller first dimension 112a of peripheral member portions 112 of members 110, the smaller gaps 94 formed between adjacent members 110 on convex side 91 of elongated element 90 upon bending thereof.

For example, Figs. 2A-2F show a rod as an example of elongated element 90, the rod having a diameter 97 of 6 mm, supported within device 100 and being bent to provide a curvature radius 98 of 45 mm in the bending region.

It is noted that such a small curvature radius 98 (e.g., of 45 mm) may be required in extreme cases during, for example, orthopedic surgeries. Such cases may, for example, include adjusting elongated element 90 (e.g., the rod) of, for example, a pedicle screw assembly to fit curvature of a spinal cord between L5-S1 spinal segments thereof.

Figs. 2A, 2B, 2C, 2D, 2E and 2F further show an example of various configurations of device 100 in which members 110 have peripheral member portions 112 with, for example, first dimension 112a of 2.0, 1.4, 1.0, 0.8, 0.6 and 0.4 mm, respectively, that provide gaps 94 on convex side 91 of elongated element 90 (upon bending thereof) of 0.31, 0.21, 0.15, 0.12, 0.09 and 0.06 mm, respectively. It is noted that Figs. 2A-2F show portions of elongated element 90 and of two adjacent members of multiple members 110 only for sake of clarity.

Second dimension 112b of peripheral member portions 112 of members 110 may have no effect (or substantially has no effect) on gaps 94 formed between adjacent members 110 on convex side 91 of bended elongated element.

Second dimension 112b may be determined to prevent overlapping of adjacent members 110 onto each other on concave side 92 of bended elongated element 90. Second dimension 112b of peripheral member portions 112 of members 110 may range between 0.05-0.3 mm.

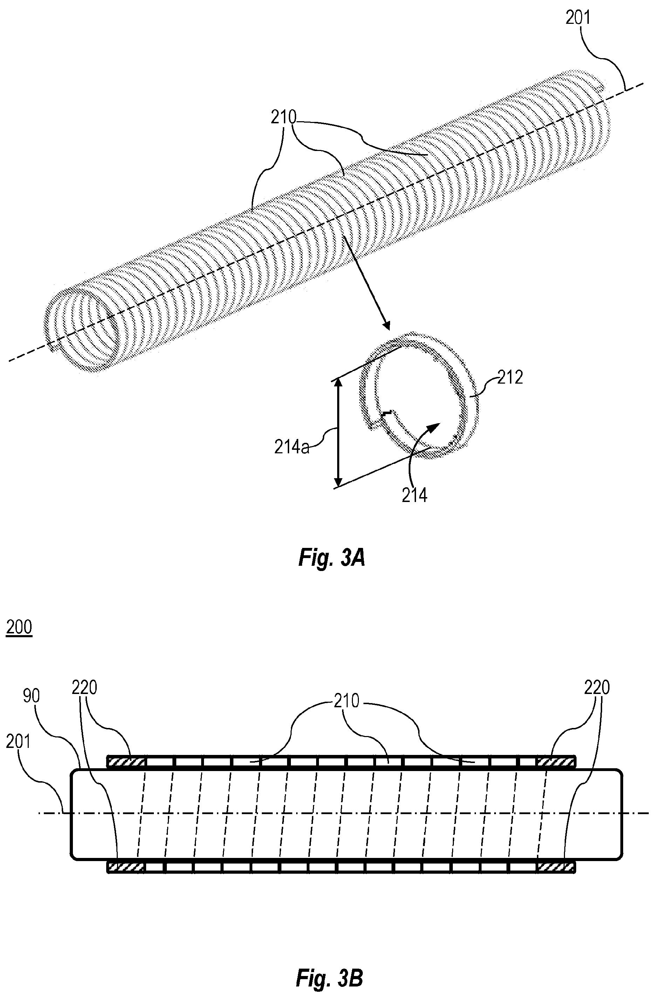

Reference is now made to **Figs. 3A** and **3B****,** which are schematic illustrations of a coiled device 200 for maintaining a shape of a transverse cross-sectional profile of an elongated element 90 useful for understanding the invention.

Coiled device 200 may include multiple coils 210 arranged along a longitudinal axis 201 of device 200, wherein adjacent coils of multiple coils 210 are helically connected with each other to form coiled device 200 (e.g., as shown in Fig. 3A).

Coils 210 of device 200 may be similar to members 110 of device 100 described above with respect to Figs. 1A-1E and Figs. 2A-2F. Coiled device 200 may be made by helically connecting adjacent members of multiple members 110 of device 100 (e.g., described above with respect to Figs. 1A-1E and Figs. 2A-2F) with each other.

Coiled device 200 may enable a desired measure of relative motion of coils 210 (e.g., of coils of device 200) with respect to each other. Coiled device 200 may enable a desired measure of deformation and/or elasticity of peripheral member portions 212 of coils 210 in a direction that is perpendicular to longitudinal axis 201 of device 200. For example, coils 210/peripheral portions 212 of coils 210 may slightly expand/collapse in the direction that is perpendicular to longitudinal axis 201.

Dimensions 214a of central member opening 214 of coils 210 of coiled device 200 may be slightly smaller (by no more than 5%) as compared to dimensions of the cross-sectional profile of elongated element 90 to be used with.

For example, if elongated element 90 is a rod having a diameter of 6 mm, then inner diameter 214a of central member opening 214 of coils 210 may be, for example 5.9 mm. Thus, coils 210 may slightly expand when receiving rod 90 and further push onto rod 90 upon receiving of rod 90 therein, thereby providing tight support of rod 90 within coiled device 200.

Stoppers 220 may removably affix coils 210 at both ends of coiled device 200 (e.g., as shown in Fig. 3B) to, for example, prevent undesired twisting/untwisting and/or axial sliding of coiled device 200 during bending. Stoppers 220 may be similar to stoppers 120 described above with respect to Figs. 1C and 1D.

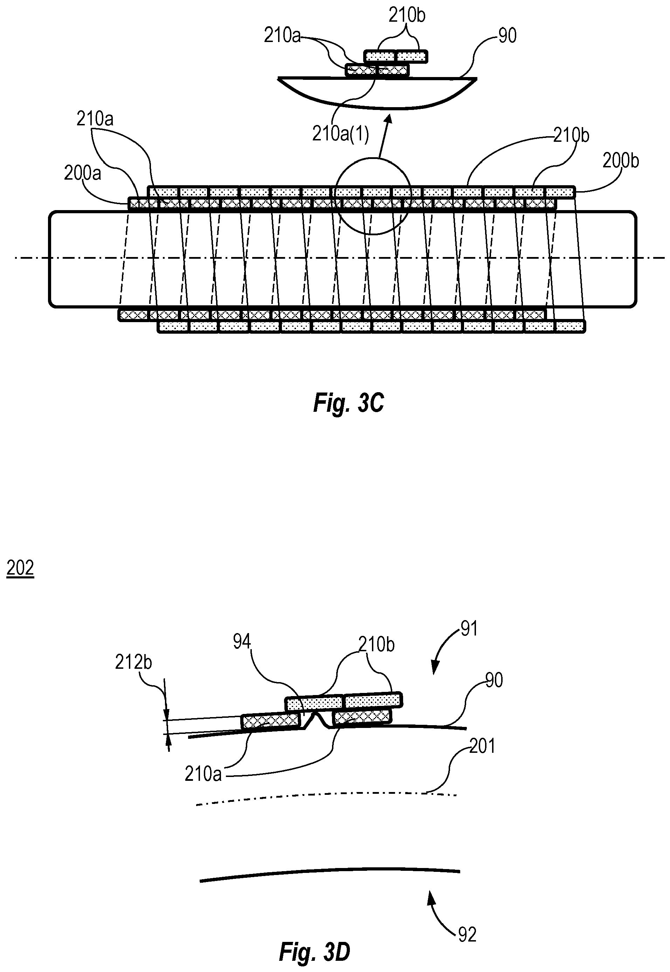

Reference is now made to **Figs. 3C** and **3D****,** which are schematic illustrations of an assembly 202 including two coiled devices 200a, 200b for maintaining a shape of a transverse cross-sectional profile of an elongated element 90 useful for understanding the invention.

More than one coiled device 200 may be used with elongated element 90. For example, a first coiled device 200a having first coils 210a and a second coiled device 200b having second coils 210b may be used (e.g., coiled devices 200a and 200b as shown in Fig. 3C).

First coiled device 200a may tightly receive and support elongated element 90 (e.g., as described above with respect to 3A-3B). Second coiled device 200b may tightly receive and support first coiled device 200a with elongated element 90 supported therein (e.g., as shown in Fig. 3C).

Second coiled device 200b may be positioned with respect to first coiled device 200a such that second coils 210b of second coiled device 200b overlap with regions of contact 210a(1) between adjacent first coils 210a of first coiled device 200a (e.g., as shown in Figs. 3C and 3D).

Such relative positioning of second coiled device 200b with respect to first coiled device 200a may, for example, limit a measure of protrusion of portions of heated elongated element 90 into gaps 94 between adjacent first coils 210a of first coiled device 200a when heated elongated element 90 is being bent. In this manner, portions of heated elongated element 90 may not protrude into gaps 94 more than second dimension 212b of peripheral member portions 212 of first coils 210a (e.g., as shown in Fig. 3D).

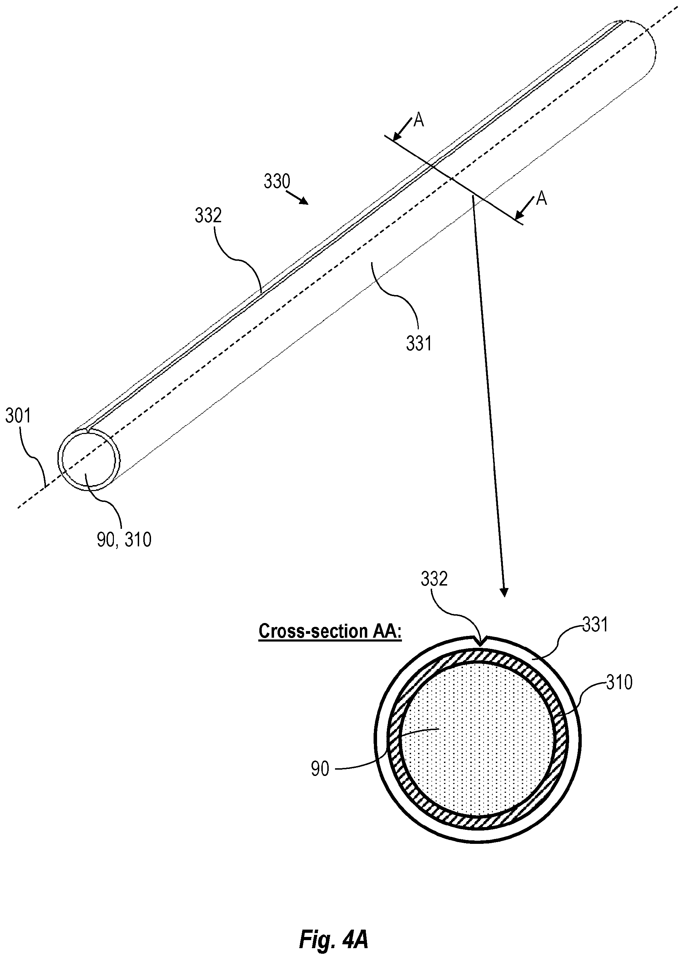

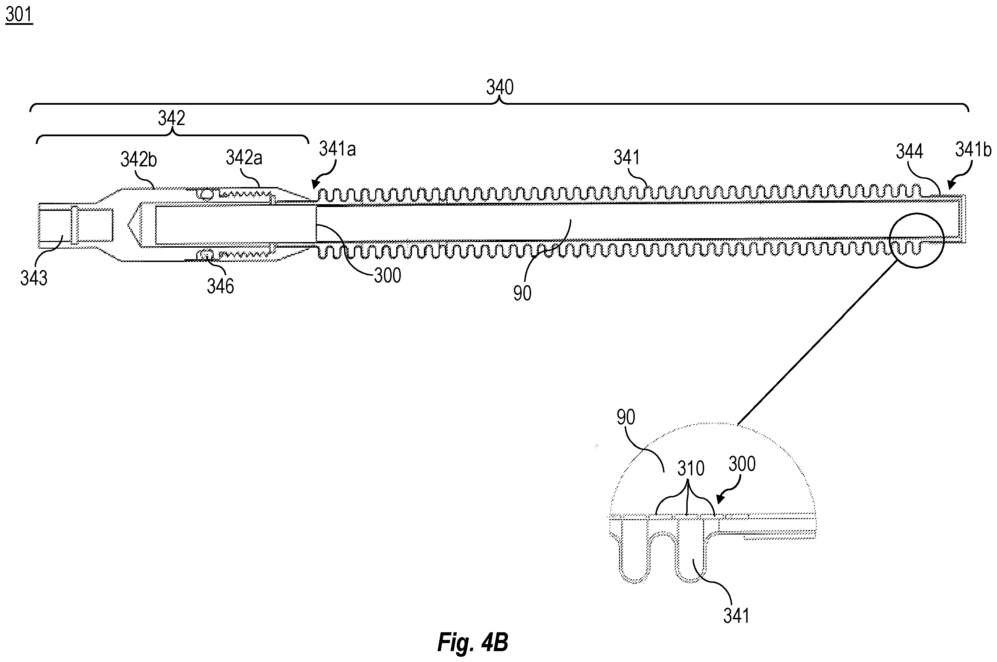

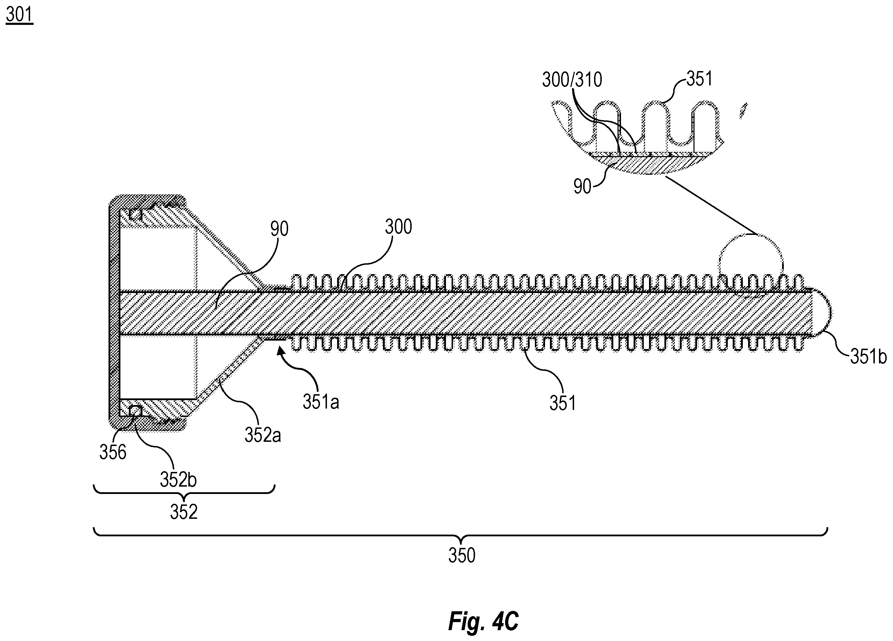

Reference is now made to **Fig. 4A****,** **4B** and **4C****,** which are schematic illustrations of an assembly 301 including a device 300 for maintaining a shape of a transverse cross-sectional profile of an elongated element 90 and an envelope 330, 340, and 350, respectively useful for understanding the invention.

Fig. 4A shows an isometric view of assembly 301 and a cross section AA of assembly 301. Figs. 4B and 4C show longitudinal cross-section views of assembly 301.

Assembly 301 may include a device 310 for maintaining a shape of a cross-sectional profile of an elongated element 90 and an envelope. Figs. 4A, 4B and 4C show schematic illustrations of a first example of an envelope 330, a second example of an envelope 340 and a third example of an envelope 350, respectively.

Device 300 may include multiple members 310 arranged along a longitudinal axis 301 of device 300. Device 300 and members 310 may be similar to device 100 and members 110, respectively, described above with respect to Figs. 1A-1E and Figs. 2A-2F and/or to device 200 and coils 210, respectively, described above with respect to Figs. 3A-3B.

The envelope may surround members 310 and elongated element 90 supported therein. The envelope may be made of an elastic material. The envelope may be made of a material that does not undergo thermal deformation at the predetermined temperature value (e.g., at 400°C). The envelope may be made of a biocompatible material. The envelope may be arranged to be released from members 310 / elongated element 90 after bending of elongated element 90.

Fig. 4A schematically shows a first example of an envelope 330. Envelope 330 may include a tube 331. Tube 331 may be made of, for example, nitinol. Tube 331 may be adapted in shape and dimensions to tightly envelope members 310 of device 300. Tube 331 may include an indent 332 along the entire (or substantially the entire) length of tube 331. The strength of tube 331 in vicinity of indent 332 may be smaller as compared to other regions of tube 331. The measure of indentation of indent 332 into walls of tube 331 may be predetermined to enable breaking or tearing and releasing of tube 331, for example, after bending of elongated element 90 and before implanting elongated element 90 into a patient's body.

Fig. 4B schematically shows a second example of an envelope 340. Envelope 340 may include a bellow tube 341. The use of a bellow tube, such as bellow tube 341, may provide an airtight cover which is flexible enough to enable bending it with its content. It will be apparent that other envelopes providing these features may be used for this purpose. Bellow tube 341 portion of envelope 340 may cover at least part of elongated element 90 when it is inserted to a rest place within bellow tube 341. Envelope 340 may be made of, for example, stainless steel. Envelope 340 may, for example, include a first cap 342 adapted to close a first end 341a of bellow tube 341 and a second cap 344 adapted to close a second end 341b of bellow tube 341.

First cap 342 may include a proximal first-cap part 342a and a distal first-cap part 342b. Proximal first-cap part 342a may be connected to first end 341a of bellow tube 341, and distal first-cap part 342b may be screwable onto proximal first-cap part 342a. Envelope 340 may include a seal 346. Seal 346 may be disposed between proximal first-cap part 342a and distal first-cap part 342b.

First cap 342, for example, distal first-cap part 342b, may include a connector 343 for a rod used to insert assembly 301 into a heating device for heating.

When distal first-cap part 342b is unscrewed from proximal first-cap part 342a, an end of elongated element 90 may protrude from proximal first-cap part 342a and may be slidably released from envelope 340.

Fig. 4C schematically shows a third example of an envelope 350. Envelope 350 may include a bellow tube 351. Envelope 350 may be made of, for example, stainless steel. Envelope 350 may, for example, include a cap 352 adapted to close a first end 351a of bellow tube 351. Bellow tube 351 portion of envelope 350 may cover at least part of elongated element 90 when it is inserted to a rest place within bellow tube 351. Second end of 351b of bellow tube 351 may be, for example, a closed end.

Cap 352 may include a first cap part 352a and a second cap part 352b. First cap part 352a may be connected to first end 351a of bellow tube 351, and second cap part 352b may be screwable onto first cap part 352a. Envelope 350 may include a seal 356. Seal 356 may be disposed between first cap part 352a and second cap part 352b.

When second cap part 352b is unscrewed from first cap part 352a, elongated element 90 may be slidably released from envelope 350.

Assembly 301 may be provided as a kit further including elongated element 90. Elongated element 90, device 300 and at least an inner portion of the envelope may be sterile. An outer portion of the envelope may be not sterile. Device 310 may be thread onto elongated element 90, and both may be inserted into the envelope. The envelope accommodating sterile elongated element 90 and sterile device 310 may be inserted into a heating device for heating of elongated element 90 and then inserted into a bending device for bending elongated element 90 while keeping elongated element 90 and device 310 sterile. Upon bending and cooling, a person having a non-sterile hand may hold the envelope and, for example, unscrew the cap thereof (e.g., as described above with respect to Figs. 4B and 4C), while a person having a sterile hand may remove sterile elongated element 90 from within the envelope. In this manner, the envelope may enable using non-sterile heating device and/or non-sterile bending device.

As described above, elongated element 90 containing thermoplastic polymer(s) should be heated or pre-heated in order to be shaped (e.g., bent). Heating/pre-heating of elongated element 90 may be done using any heating means known in the art. For example, elongated element 90 may be heated using laser, infrared radiation, or induction or resistant prior to positioning of elongated element 90 into a bending device or upon positioning of elongated element 90 in the bending device.

Elongated element 90 may be disposed within a cross-sectional profile maintaining device prior to heating and bending thereof (e.g., device 100, 200, as described above with respect to Figs. 1A-1E and 2A-2F, Figs. 3A-3B, respectively). Elongated element 90 with the cross-sectional profile maintaining device may be disposed within an envelope prior to heating and bending thereof (e.g., envelope 330, 340, 350 described above with respect to Figs. 4A, 4B, 3C, respectively).

For sake of clarity, the description below made with respect to Figs. 5, 6A-6C, 7A-7E, 8A-8E, 9A-9B, and 10A-10C refers to a "elongated assembly" or "elongated assembly 80", wherein elongated assembly 80 may include at least one of: elongated element 90, elongated element 90 removably disposed within the cross-sectional profile maintaining device and the envelope removably accommodating elongated element 90 disposed within the cross-sectional profile maintaining device.

Reference is now made to **Fig. 5****,** which is a schematic illustration of a heating device 400 for heating an elongated assembly useful for understanding the invention.

Heating device 400 may include a housing 410 and at least one heating element 420 that at least partly envelopes housing 410.

Housing 410 is an annular (or substantially annular) body (e.g., as shown in Fig. 5). Housing 410 may have an interior 412 that is arranged to receive and accommodate the elongated assembly. It is noted that the elongated assembly is not shown in Fig. 5 for sake of clarity.

At least one heating element 420 may have a helical shape and envelopes housing 410 along the entire (or substantially entire) length of housing 410. For example, heating device 400 may include three heating elements: a first heating element 422, a second heating element 444 and a third heating element 426 (e.g., as shown in Fig. 5). Use of more than one heating element may enable heating of the elongated assembly, even when one of heating elements fails.

Heating element(s) 420 may be low voltage, optionally battery-operated. For example, heating element(s) 420 may be operated by 48 Volt DC battery.

Heating device 400 may be sterile. For example, heating device 400 may be made of materials that enable sterilization of the entire heating device (e.g., including housing and heating element set 420) before/after each operational procedure.

Heating device 400 may not require sterilization.

Elongated assembly may include the envelope accommodating elongated element disposed within the cross-sectional profile maintaining device, wherein the envelope may keep elongated element 90 sterile during heating and/or bending thereof (e.g., as described above with respect to Figs. 4A-4C).

Heating device 400 may include a heating envelope 430 (e.g., as shown in Fig. 5). Heating envelope 430 may be made of a material that may be sterilized (e.g., stainless steel).

Heating envelope 430 may include a first heating envelope end 431, a second heating envelope end 432 and a heating element interior 433. Heating envelope 430 may include an opening 431a on, for example, first heating envelope end 431 and may, for example, be closed at second heating envelope end 432.

Heating envelope 430 may receive, through opening 431a, and may accommodate within heating envelope interior 433, the elongated assembly. Heating envelope 430 with the elongated assembly may be positioned within a housing interior 412 of housing 410. Heating device 400 may be adapted to supply a desired measure of heat energy though heating element(s) 420 to elevate the temperature of the elongated assembly and particularly of elongated element 90 disposed therein up to the predetermined temperature value (e.g., 400°C).

In this manner (e.g., using envelope 330, 340, 350 or heating envelope 430), the elongated assembly and particularly elongated element 90 disposed therein may be heated to the predetermined temperature value while eliminating a need in sterilizing heating device 400 before/after each operational procedure.

The elongated assembly and heating envelope 430 may be part of a kit.

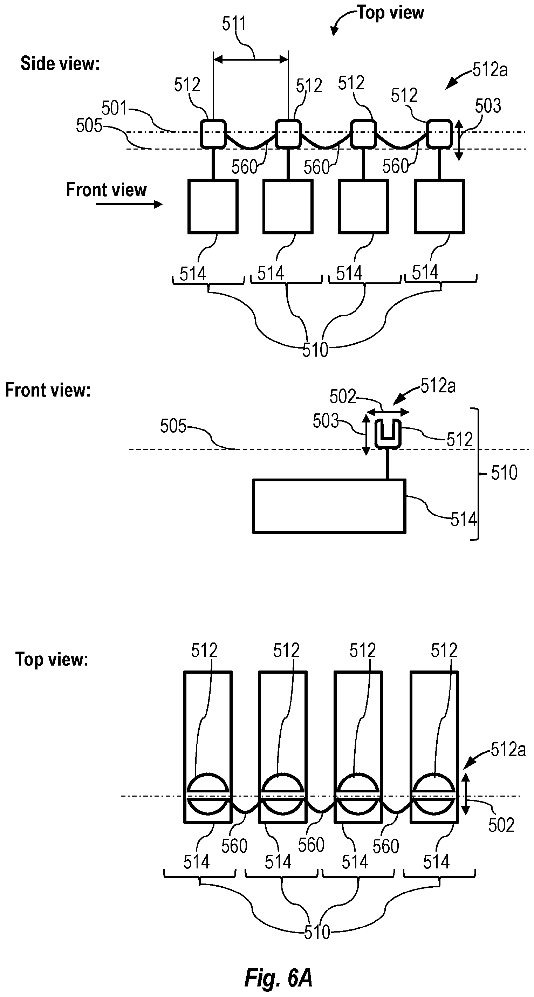

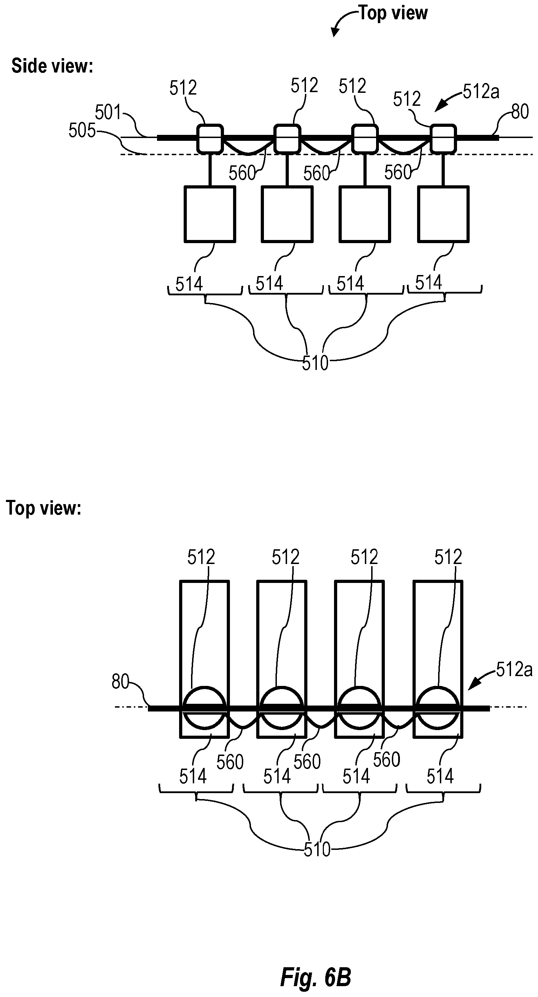

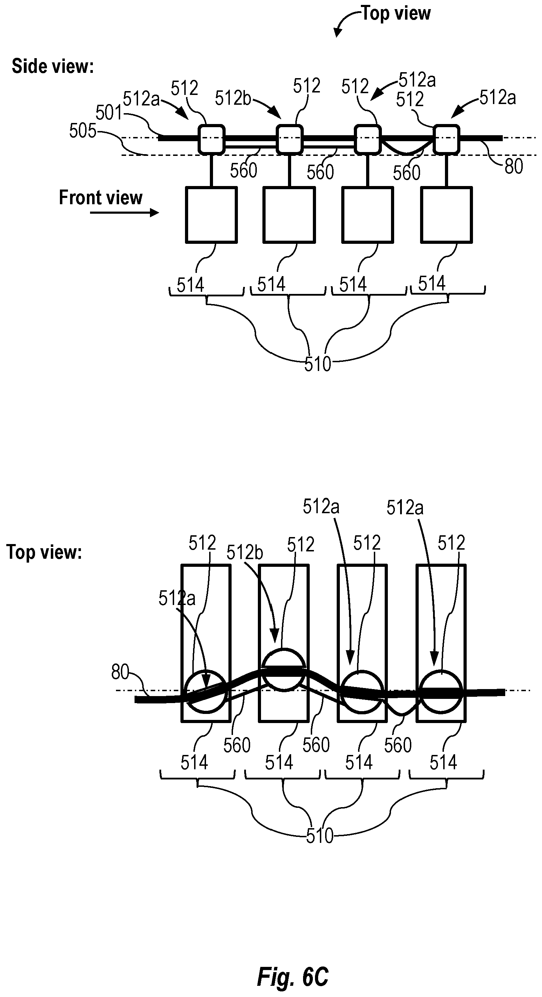

Reference is now made to **Figs. 6A****,** **6B** and **6C****,** which are schematic illustrations of a bending device 500 for bending an elongated assembly 80 useful for understanding the invention.

Bending device 500 may include multiple bending units 510 (e.g., as shown in Fig. 6A). Bending units 510 may be positioned at a distance 511 with respect to each other (e.g., as shown in Fig. 6A). Distance 511 between adjacent bending units 510 may range between 10-20 mm. A number of bending units 510 may range between 10-30. It is noted that Figs. 6A, 6B and 6C show four (4) bending units 510 for sake of clarity.

Each of bending units 510 may include a receiving member 512 and a driving assembly 514 (e.g., as shown in Fig. 6A).

Each of receiving members 512 of bending units 510 may be movable between an initial position 512a (e.g., as shown in Figs. 6A and 6B) and at least one other position 512b (e.g., as shown in Fig. 6C).

Receiving members 512 may be aligned along a longitudinal axis 501 of bending device 500 in a plane 505 when all receiving members 512 are in their initial positions 512a (e.g., as shown in Figs. 6A and 6B). Receiving members 512 of bending units 510 may be arranged to receive, when in initial position 512a, corresponding portions of elongated assembly 80 (e.g., as shown in Fig. 6B). It is noted that elongated assembly 80 is not shown in Fig. 6A for sake of clarity only.

Each of receiving members 512 may be movable in a first direction 502 and/or in a second direction 503 that is perpendicular to longitudinal axis 501 when moved between its respective initial position 512a and at least one other position 512b (e.g., as shown in Fig. 6A). For example, driving assemblies 514 of bending units 510 may be arranged to move respective receiving members 512 between their respective initial and at least one other positions 512a, 512b, respectively.

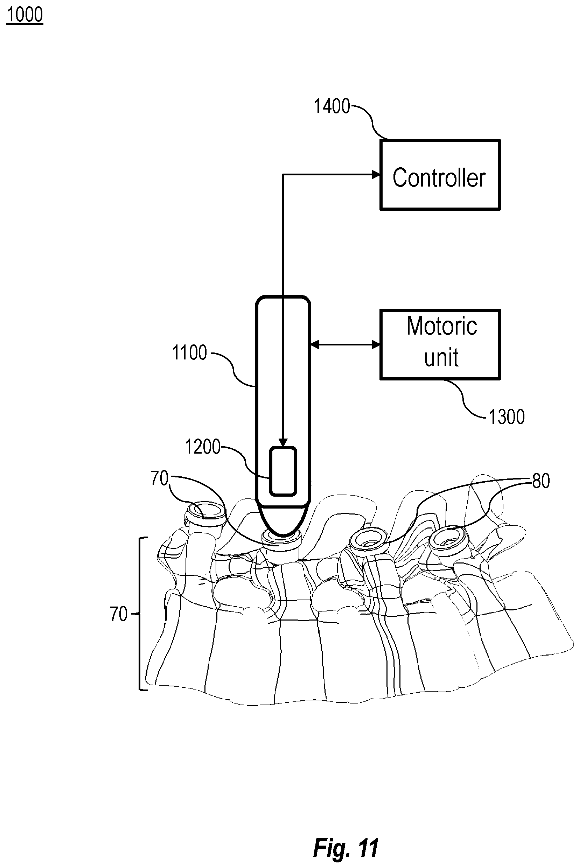

The direction of movement and/or the measure of movement and/or at least one other position 512b of each of receiving members 512 may be determined based on, for example, a desired bending profile of elongated element 90 disposed within elongated assembly 80. The desired bending profile of elongated element 90 may be determined/predetermined based on, for example, an anatomy of an implantation site of elongated element 90 (e.g., as described below with respect to Fig. 11).

Elongated assembly 80 may be pre-heated (e.g., using heating device 400 as described above with respect to Fig. 5) prior to positioning thereof in bending device 500. Alternatively, elongated assembly 80 may be heated upon positioning thereof in bending device 500 using, for example, laser or infrared radiation.

Upon positioning of elongated assembly 80 in bending device 500, receiving member 512 of at least one of bending units 510 may be moved from its initial position 512a to one of its other positions 512b in one or two directions 502, 503, based on the predetermined bending profile, thereby bending elongated assembly 80 and elongated element 90 disposed therein into a desired shape. For example, Fig. 6C shows receiving member 512 of one of bending units 510 in one of its other positions 512b and receiving members 512 of other bending units 510 in their initial position 512a.

Receiving members 512 of bending units 510 may rotate (e.g., about an axis perpendicular to plane 505) to provide smooth bending of elongated assembly 80 and elongated element 90 disposed therein (e.g., as shown in Fig. 6C).

In this manner, elongated element 90 (e.g., disposed within elongated assembly 80) containing thermoplastic polymer(s) may be bent according to the predetermined bending profile while preventing distortion of the shape of the cross-sectional profile of elongated element 90 in the bending regions thereof.

Bending device 500 may include connectors 560. Connectors 560 may be, for example, deformable wires or ropes. Each of connectors 560 may be connected at its end to receiving members 512 of adjacent bending units 510 (e.g., as shown in Figs. 6A-6C). Connectors 560 may limit the measure of movement of adjacent receiving members 512 with respect to each other, thereby limiting a bending radius of elongated element 90 in the bending region thereof. The length of connectors 560 may be determined to ensure that the bending radius of elongated element 90 in the bending region will not exceed a predetermined bending radius value. The predetermined bending radius value may be 45 mm.

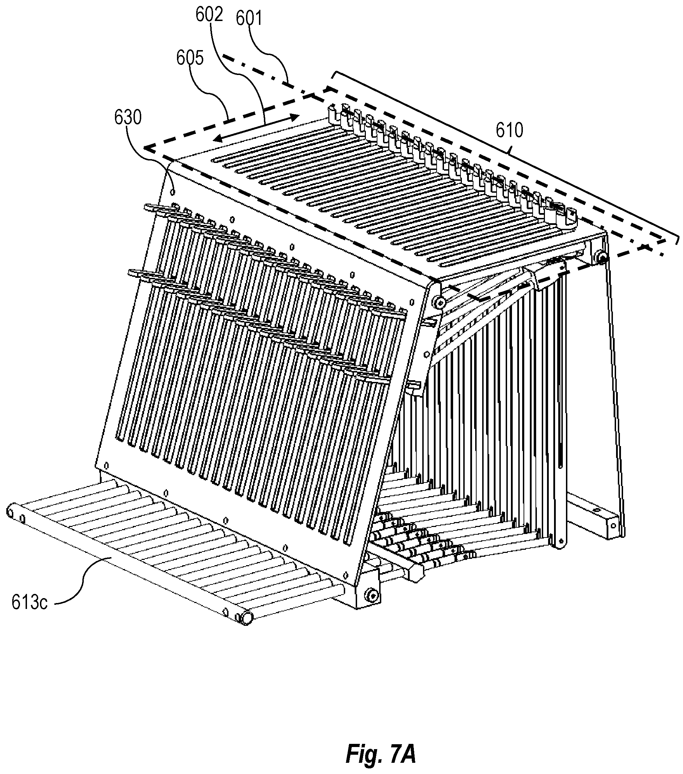

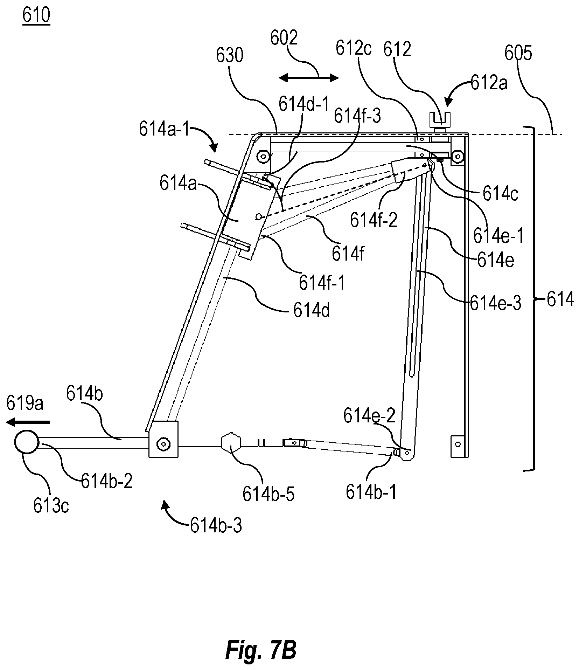

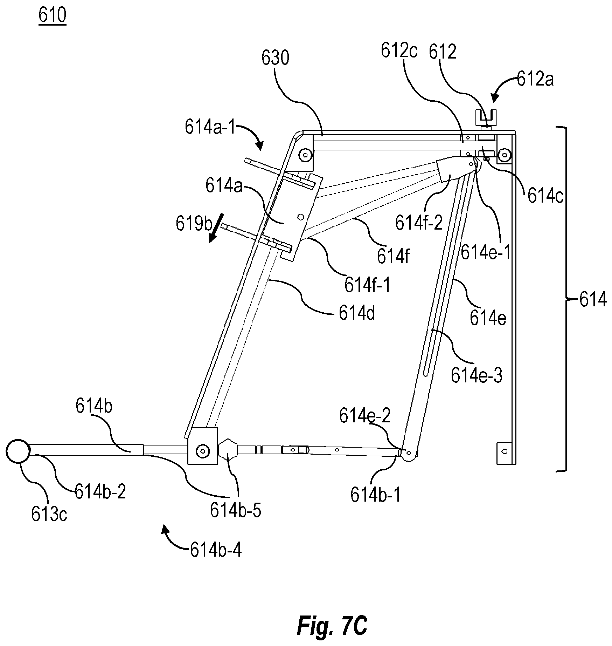

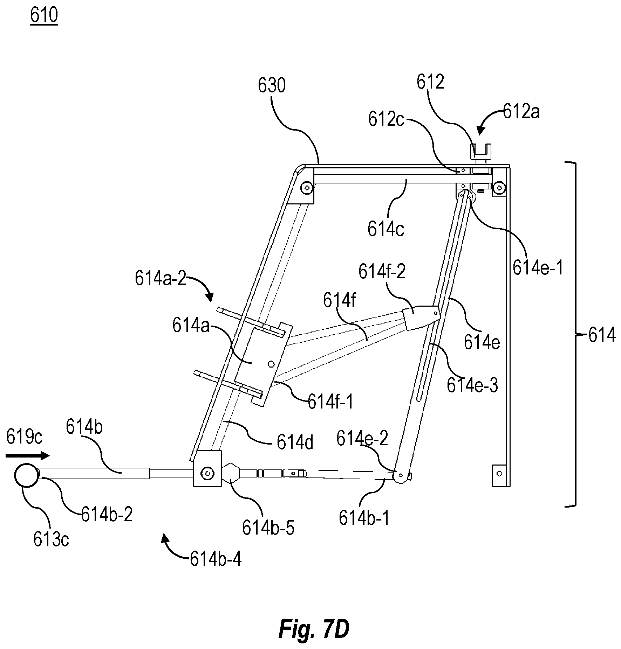

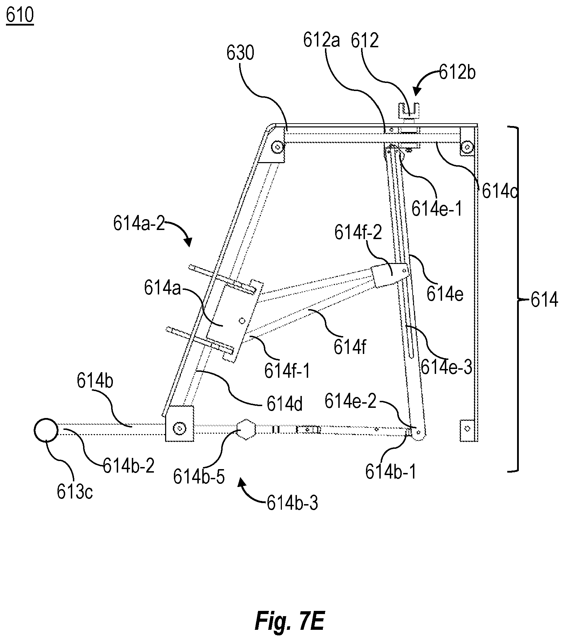

Reference is now made to **Figs. 7A****,** **7B****,** **7C****,** **7D** and **7E****,** which are schematic illustrations of a bending device 600 for bending an elongated assembly, including bending units 610 with mechanically adjustable driving assemblies 614 useful for understanding the invention.

Fig. 7A shows a perspective view of bending device 600, and Figs. 7B, 7C, 7D and 7E show a side view of a single bending unit 610.

Bending device 600 may include a housing 630 arranged to accommodate multiple bending units 610 of bending device 600 (e.g., as shown in Fig. 7A).

Each of bending units 610 may include receiving member 612 and driving assembly 614 coupled to a respective receiving member 612 and arranged to move respective receiving member 612 based on the predetermined bending profile of elongated element 90.

Bending units 610 (e.g., receiving members 612 and driving assemblies 614) of device 600 may be similar to bending units 510 (e.g., receiving members 512 and driving assemblies 514) as described above with respect to Figs. 6A-6C.

Figs. 7B, 7C, 7D and 7E show single bending unit 610 for sake of clarity. It is noted that other bending units 610 may have similar structure and operate in similar way.

Driving assembly 614 of each of bending units 610 may include a mover 614a and an actuation arm 614b.

Mover 614a of each of bending units 610 may be movable between an initial mover position 614a-1 and at least one another mover position 614a-2 and arranged to define/dictate the measure movement of respective receiving member 612, upon actuation by respective actuation arm 614b (e.g., as described below with respect to Figs. 7B, 7C, 7D and 7E).

Each of driving assemblies 614 of each of bending units 610 may include a first rail 614c, a second rail 614d, a rotatable arm 614e and a rotational axis arm 614f (e.g., as shown in Figs. 7B, 7C, 7D and 7E).

First rail 614c of each of driving assemblies 614 may be coupled at its ends to housing 630 in a plane that is parallel to plane 605 and in first direction 602 (e.g., as shown in Fig. 7B, 7C, 7D and 7E).

Receiving member 612 of each of bending units 610 may be coupled at its end 612c to respective first rail 614c and arranged to move along respective first rail 614c between its respective initial position 612a and at least one other position 612b.

Rotatable arm 614e of each of driving assemblies 614 may be pivotally coupled at its first end 614e-1 to an end 612c of respective receiving member 612 and may be pivotally coupled at its second end 614e-2 to a first end 614b-1 of respective actuation arm 614b.

A second end 614b-2 of actuation arm 614b of each of driving assemblies 614 may extend from housing 630. Second ends 614b-2 of actuation arms 614b of driving assemblies 614 of all bending units 610 are connected using an actuation rod 613c.

Actuation arm 614b of each of driving assemblies 614 may be movable (e.g., in a plane parallel to plane 605 and in first direction 602) between its initial position 614b-3 and extended position 614b-4.

Actuation arm 614b of each of driving assemblies 614 may include one or more stoppers 614b-5 to restrict motion of actuation arm 614b between its initial position 614b-3 and extended position 614b-4.

Actuation arms 614b of all driving assemblies 614 of all bending units 610 may be simultaneously moved, for example, by manually pushing/pulling actuation rod 613c (with respect to housing 630) that connects second ends 614b-2 of actuation arms 614b of driving assemblies 614.

Second rail 614d of each of driving assemblies 614 may be coupled at its ends to housing 630 and may be aligned parallel to rotatable arm 614e when respective actuation arm 614b is fully extended (e.g., as shown in Fig. 7C).

Mover 614a of each of driving assemblies 614 may be movably coupled to second rail 614d and may be arranged to move along second rail 614d between its respective initial position 614a-1 and respective at least one another position 614a-2.

Mover 614a of each of driving assemblies 614 may be moved manually by the user of bending device 600. Mover 614a of each of driving assemblies 614 may include an affixing mechanism (e.g., spring-loaded affixing mechanism) that may enable motion of the mover when, for example, pressed, and hold the position of the mover when released.

Rotational axis arm 614f of each of driving assemblies 614 may be coupled at its first end 614f-1 to respective mover 614a and movably coupled at its second end 614f-2 to respective rotatable arm 614e of respective driving assembly 614.

Second end 614f-2 of rotational axis arm 614f of each of driving assemblies 614 may be arranged to at least partly embrace respective rotatable arm 614e and to move within dents 614e-3 on respective rotational arm 614e. Dents 614e-3 of rotational arm 614e of each of driving assemblies 614 may initiate at first end 614e-1 of rotational arm 614e and may extend towards second end 614e-2 of rotational arm 614e along at least a portion and along at least one of sides of rotational arm 614e.

A position of second end 614f-2 of rotational axis arm 614f of each of driving assemblies 614 (dictated by positions 614a-1, 614a-2 of respective mover 614a) may define a rotational axis about which respective rotational arm 614e will rotate upon actuation by respective actuation arm 614b.

For example, movement of actuation arm 614b of each of driving assemblies 614 between its extended and initial positions 614b-4, 614b-3 may move second end 614e-2 of respective rotatable arm 614e, thereby rotating respective rotatable arm 614e about the rotational axis defined by second end 614f-2 of respective rotational axis arm 614f and moving respective receiving member 612 along respective first rail 614c into one of its other positions 612b (e.g., as shown in Fig. 7E).

The length of first rail 614c, the length of rotational axis arm 614f, angle 614d-1 and angle 614f-3 of each of driving assemblies 614 may be determined to ensure that second end 614f-2 of respective rotational axis arm 614f is adjacent to first end 614e-1 of respective rotatable arm 614e when respective mover 614a is in its initial position 614a-1, thereby ensuring that respective receiving member 612 remains in its initial position 612a independent of movement of respective actuation arm 614b.

The length of first rail 614c, the length of rotational axis arm 614f, angle 614d-1 and angle 614f-3 of each of driving assemblies 614 may be determined to ensure that respective rotatable arm 614e is parallel (or substantially parallel) to respective second rail 614d when respective actuation arm 614b in in its extended position 614b-4.

An offset of the rotational axis of rotational arm 614e of each of driving assemblies 614 with respect to first end 614e-1 defined by the position of second end 614f-2 of respective rotational axis arm 613d, which in turn is defined by the position 614a-2 of respective mover 614a, dictates/defines the measure of movement of respective receiving member 612 with respect to its initial position 612a. The larger the offset of the rotational axis of rotational arm 614e, the larger the measure movement/displacement of receiving member 612.

In this manner, position 614b-2 of movers 614b of bending units 610 dictate/define the measure of movement of respective receiving members 612.

Bending device 600 may be easily pre-set in order to bend the elongated assembly and particularly elongated element 90 disposed therein into the desired shape. The description below (yet made with respect to Figs. 7B-7E) provides an exemplary method of pre-setting of bending device 600.

At a first stage, actuator arms 614b of all driving assemblies 614 of all bending units 610 may be moved from their initial positions 614b-1 to their extended positions 614b-2. This may be done by, for example, pulling actuation rod 613c (e.g., that connects actuator arms 614b of driving assemblies 614 of all bending units 610) away from housing 630 of bending device 600 (e.g., as indicated by arrow 619a in Fig. 7B).

At the next stage, the position of movers 614a of each of driving assemblies 614 of each of bending units 610 may be set to define/dictate the measure of movement of receiving members 612 of respective bending units 610, based on the desired bending profile of elongated element 90 (e.g., as indicated by arrow 619b in Fig. 7C).

For example, movers 614a of driving assemblies 614 of some of bending units 610 may be moved from their initial positions 614a-1 to one of their other positions 614a-2 to define the measure of movement of receiving members 612 of the respective some bending units thereof upon actuation. Yet, in this example, movers 614a of driving assemblies 614 of other bending units 610 may remain at their initial positions 614a-1 in order to ensure that receiving members 612 of the respective other bending units thereof will remain stationary in their initial positions 612a upon the actuation.

Scale markings may be made on housing 630 adjacent to movers 612a of bending units 610 in order to enable easy operation and setting of movers' 612a position.

At the next stage, heated elongated assembly may be positioned within receiving members 612 of bending units 610. The elongated assembly is not shown in Figs. 7A-7E for sake of clarity.

Alternatively, the elongated assembly may be heated upon positioning thereof within bending device 600, for example using infrared or laser radiation.

At the next stage, actuation rod 613c that connects actuation arms 614b of all driving assemblies 614 of all bending units 610 may be pushed toward housing 630 of bending device 600 (e.g., as indicated by arrow 619c in Fig. 7D), thereby adjusting the position of receiving members 612 of all bending units 610 as defined/dictated by the positions of movers 614a of respective bending units 610 and bending the elongated assembly and particularly elongated element 90 disposed therein into the desired shape (e.g., as shown in Fig. 7E).

In this manner, bending device 600 may be easily pre-set to bend the elongated assembly and elongated element 90 disposed therein into the desired bending shape.

The entire bending device 600 (e.g., described above with respect to Figs. 7A-7E) may be sterilized before/after each operational procedure.

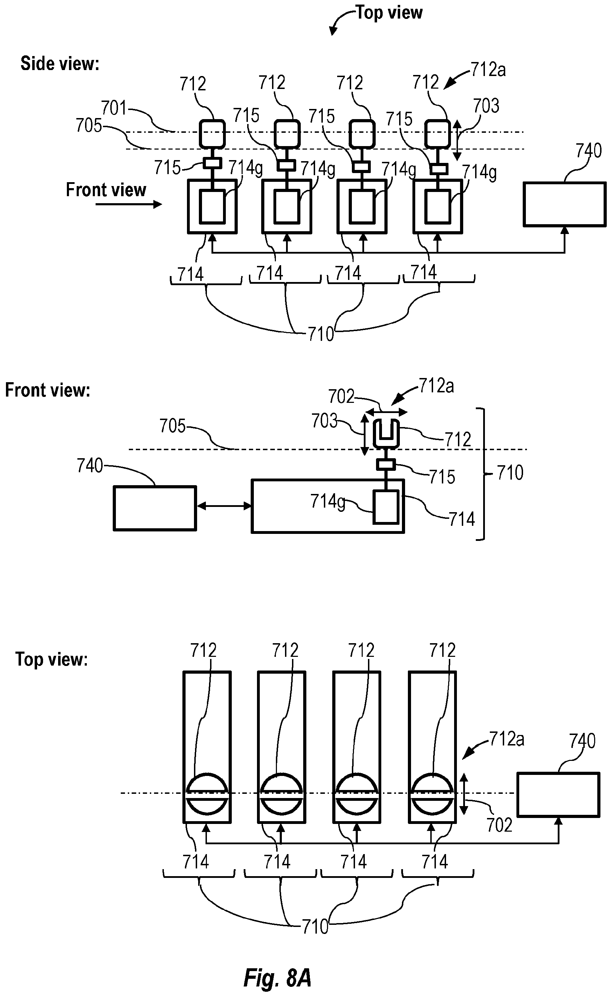

Reference is now made to Figs. 8A, which is a schematic illustration of a bending device 700 for bending an elongated assembly with bending units 710 having motorized driving assemblies 714 useful for understanding the invention.

Bending device 700 may include multiple bending units 710 and a controller 740 (e.g., as shown in Fig. 8A).

Each of bending units 710 may include receiving member 712 arranged to receive a portion of an elongated assembly and motorized driving assembly 714 arranged to move receiving member 712 of respective bending unit 710 between its respective initial position 712a and at least one other position 712b based on the desired bending profile of elongated element 90.

Bending units 710 (e.g., receiving members 712 and driving assemblies 714) of device 700 may be similar to bending units 510 (e.g., receiving members 512 and driving assemblies 514) as described above with respect to Figs. 6A-6C.

Each of driving assemblies 714 of each of bending units 710 may include at least one motor 714g (e.g., as shown in Fig. 8A). For example, motor 714g may be rotating motors. Receiving member 712 of each of bending units 710 may be coupled to motor 714g of respective driving assembly 714 using, for example, a transmission sub-unit 715. Transmission sub-unit 715 of each of bending units 710 may be arranged to, for example, transmit rotations generated by motor 714g of respective driving assembly 714 into translational movement of receiving member 712 of respective bending unit 710.

Controller 740 may be coupled to driving assemblies 714 of all bending units 710 and may be configured to control the operation of driving assemblies 714 and to thus control the movement/displacement of receiving members 712 of all bending units 710 in first direction 702 and/or in second direction 702 based on the desired bending profile.

Controller 740 may be configured to limit the measure of movement of adjacent receiving members 712 with respect to each other, thereby limiting the bending radius of the elongated assembly and elongated element 90 disposed therein in the bending region thereof and ensuring that the bending radius does not exceed the predetermined minimum bending radius value (e.g., 45 mm).

Motors 714g may be sterilizable motors. In this manner, the entire bending device 700 (e.g., described above with respect to Fig. 8A) may be sterilized before/after each operational procedure.

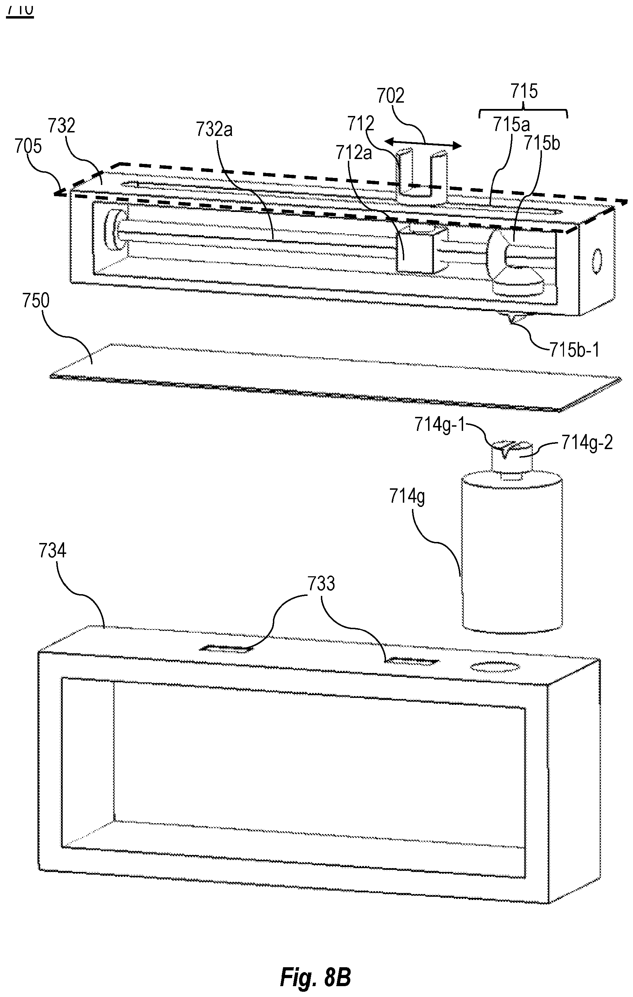

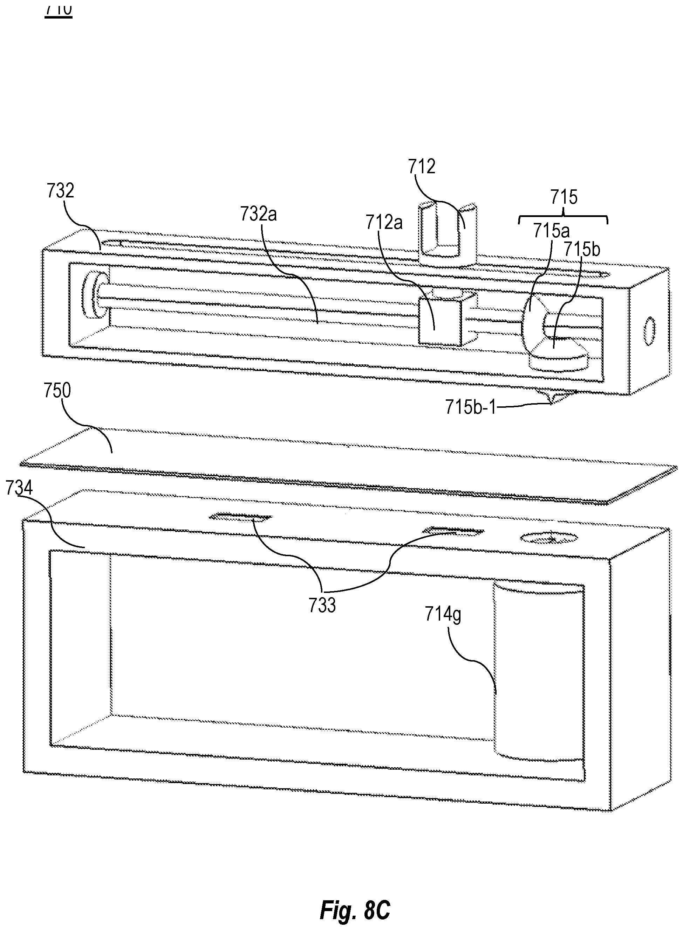

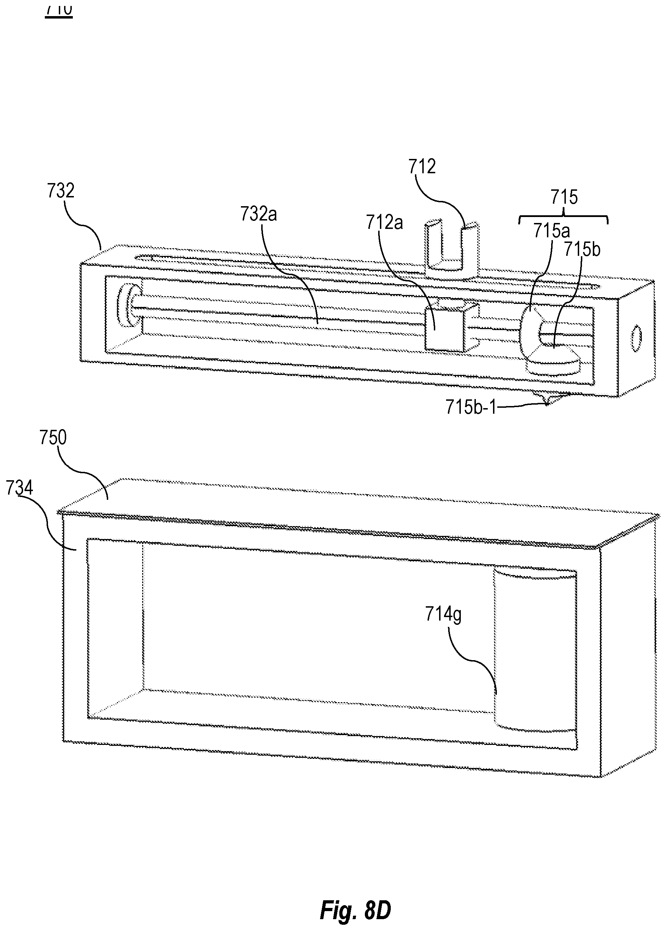

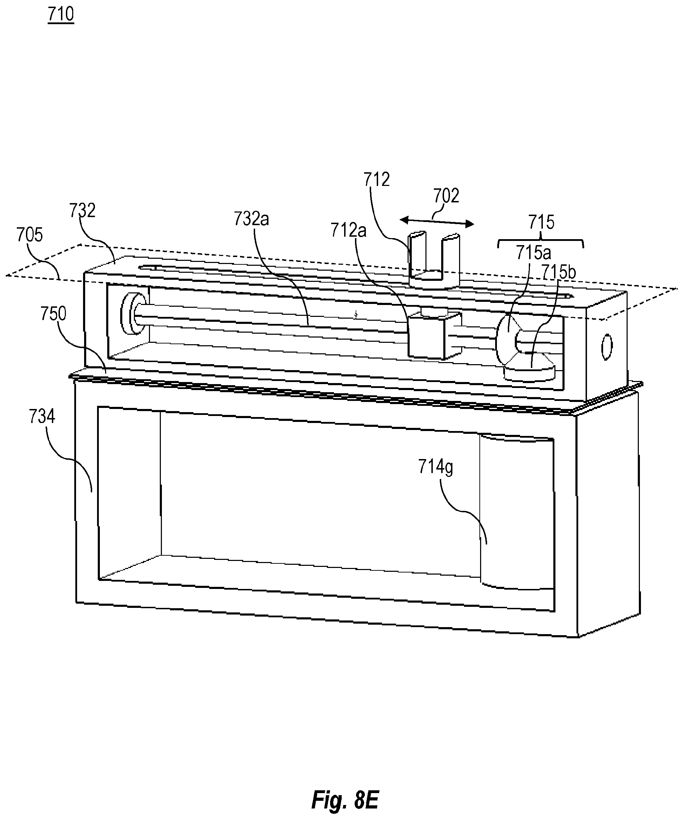

Reference is now made to Figs. **8B****,** 8C, **8D** and 8E which are schematic illustrations of a single motorized bending unit 710 of a bending device 700 for bending an elongated assembly useful for understanding the invention.

Fig. 8B shows an exploded view of bending unit 710. Fig. 8C and Fig. 8D show disassembled views of bending unit 710. Fig. 8E shows an assembled view of bending unit 710.

Bending device 700 may include a receiving members housing 732 arranged to accommodate receiving members 712 of all bending units 710 and a driving assemblies housing 734 arranged to accommodate driving assemblies 714 of all bending units (e.g., as shown in Figs. 8B-8E). Figs. 8B-8E show single bending units 710 for sake of clarity. It is noted that other bending units 710 may have a similar structure and operate in a similar way.

Receiving members housing 732 and driving assemblies housing 734 may be detachably couplable. At least one of receiving members housing 732 and driving assemblies housing 734 may include at least one magnet 733 (e.g., as shown in Figs. 8B and 8C). Magnets 733 may ensure tight contact/coupling of receiving members housing 732 with driving assemblies housing 734 yet enabling easy detachment thereof.

Each of bending units 710 may include a rod 732a attached at its end within receiving member housing 732 parallel to plane 705 and aligned in first direction 702 (e.g., as shown in Figs. 8B-8E). Receiving member 712 of each of bending units 710 may be movably coupled to respective rod 732a and arranged to move along respective rod 732 in first direction 702. Each of rods 732a may include a thread along the entire length of rods 732a.

Transmission sub-unit 715 of each of bending units 710 may be located in receiving members housing 732. Transmission unit 715 of each of bending units 710 may, for example, include a first gear 715a mounted on respective third rail 732a and a second gear 715b perpendicular to first gear 715a and detachably couplable to motor 714g of respective driving assembly 714 (e.g., as shown in Figs. 8B-8E).

For example, second gear 715b of transmission sub-unit 715 of each of bending units 710 may include a protrusion 715b-1 and motor 714g of respective driving assembly 714 may include a mating indent 714g-1 on a motor-connector 714g-2. Protrusion 715b-1 of second gear 715b of transmission sub-unit 715 of each of bending units 710 may enter indent 714g-1 of motor 714g of respective driving assembly 714 when receiving members housing 732 is coupled to driving assemblies housing 734.

Transmission sub-unit 715 of each of bending units 710 may be arranged to convert rotations generated by motor 714g of respective driving assembly 714 into translation movement of receiving member 712 of respective bending unit 710 in first direction 702. For example, transmission sub-unit 715 of each of bending units 710 may convert rotations generated by motor 714g of respective driving assembly 714 into rotation of respective rod 732a and result in movement of respective receiving member 712 in first direction 702.

Bending device 700 may include a covering 750. Covering 750 may be, for example, sterile plastic layer and/or fabric and may be arranged to cover driving assemblies housing 734 prior to coupling of receiving members housing 732 thereto. Protrusion 715b-1 of second gear 715b of transmission sub-unit 715 of each of bending units 710 may have a desired measure of sharpness to cut through covering 750 when receiving members housing 732 is being coupled to driving assemblies housing 734.

Driving assemblies 714 (e.g., motors 714g) and driving assemblies housing 734 need not to be sterilized, while receiving members 712 and receiving members housing 732 may be sterilized before/after each operational procedure.

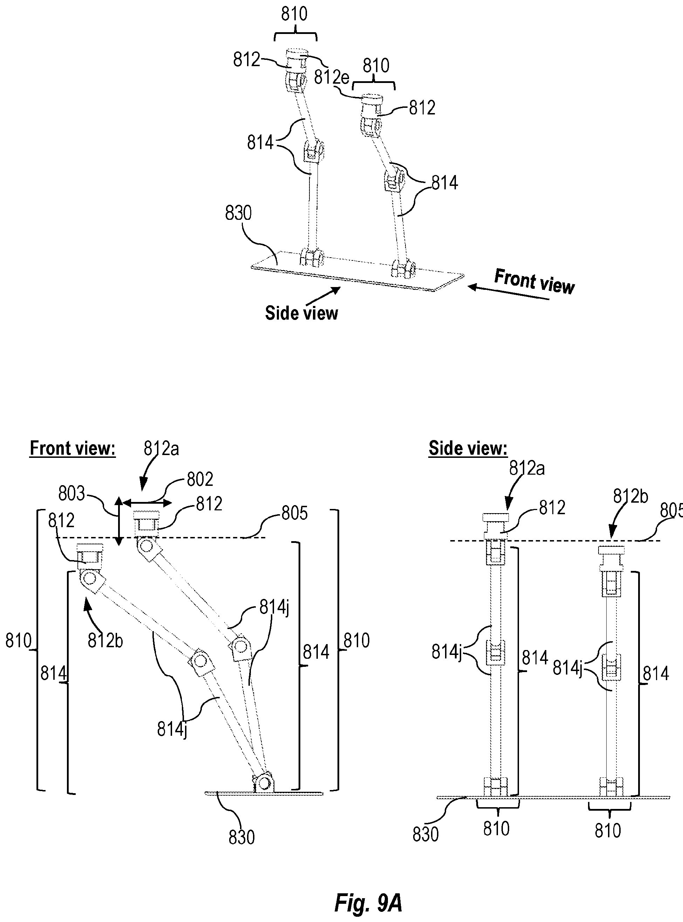

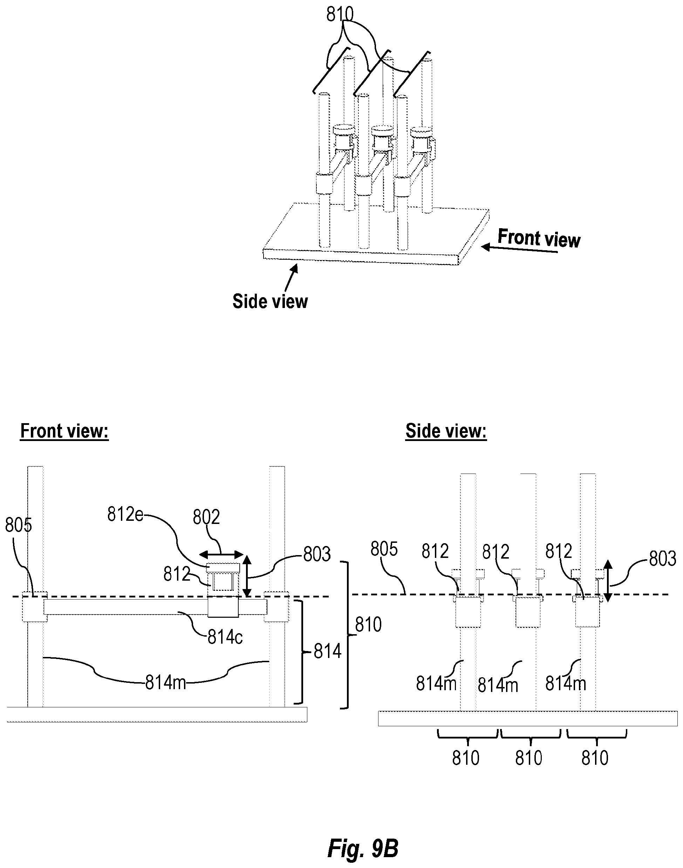

Some examples of bending devices, for example those described above with respect to Figs. 7A-7E and 8B-8E, are capable of bending the elongated assembly and particularly elongated element 90 in a single plane (e.g., planes 605, 705, respectively) and thereby provide two-dimensional bending of elongated element 90. Bending devices may be capable of bending the elongated assembly and/or elongated element 90 in more than one plane and thus providing three-dimensional bending of elongated element 90 (e.g., as described below with respect to Figs. 9A and 9B).

Reference is now made to Figs. 9A and **9B****,** which are schematic illustrations of a bending device 800 capable of providing three-dimensional bending of an elongated assembly useful for understanding the invention.

Bending device 800 may include multiple bending units 810. Each of bending units 810 may include receiving member 812 arranged to receive a portion of the elongated assembly and driving assembly 814 arranged to move receiving member 812 of respective bending unit 810 between its respective initial position 812a and at least one other position 812b based on the desired bending profile of elongated element 90.

Bending units 810 (e.g., receiving members 812 and driving assemblies 814) of device 800 may be similar to bending units 510 (e.g., receiving members 512 and driving assemblies 514) as described above with respect to Figs. 6A-6C.

Driving assemblies 814 of bending units 810 may be arranged to move respective receiving members 812 in both first direction 802 (e.g., parallel to plane 805) and in second direction 803.

Driving assemblies 814 of each of bending units 810 may include an articulated arm 814j (e.g., as shown in Fig. 9A). Articulated arm 814j of each of driving assembly 814 may be pivotally coupled at its first end to housing 810 and may be pivotally coupled at its second end to receiving member 812 of respective bending unit 810. Articulated arm 814j of each of driving assemblies 814 may enable movement of respective receiving member in first direction 802 and/or in second direction 803 (e.g., as shown in Fig. 9A).

Driving assembly 814 of each of bending units 810 may include a pair of rods 814m (e.g., as shown in Fig. 9B). Rods 814m of each of driving assemblies 814 may be coupled to housing 830 and extend in second direction 803 that is perpendicular to plane 805.

Receiving member 812 of each of bending units 810 may be movably coupled to first rail 814c of respective driving assembly 814 and arranged to move along respective first rail 814c in first direction 802. First rail 814c of each of driving assemblies 814 may be movably coupled at its ends to respective rods 814m and may be arranged to move along respective rods 814m in second direction 803. In this manner, receiving member 812 of each of bending units 810 may be moved in both first direction 802 and second direction 803.

Each of receiving members 812 may include a cap 812e arranged to cover respective receiving member 812. Caps 812e may be arranged to prevent escaping of the elongated assembly from receiving members 812 during bending.

Each of driving assemblies 814 of each of bending units 810 described above with respect to Figs. 9A and 9B may be manually adjustable (e.g., as described above with respect to Figs. 7A, 7B, 7C, 7D and 7E) or motorized (e.g., as described above with respect to Figs. 8A, 8B, 8C, 8D and 8E).

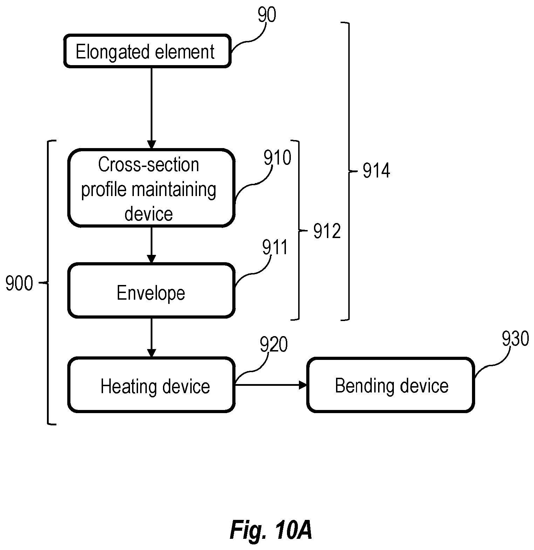

Reference is now made to Fig. 10A, which is a block diagram of a system 900 for bending an elongated assembly useful for understanding the invention.

System 900 may include a device 910 for maintaining a cross-sectional profile of elongated element 90, a heating device 920 and a bending device 930. System 900 may include an assembly 912 including device 910 and an envelope 911.

Device 910 may receive and tightly support elongated element 90 and prevent distortion of elongated element 90 during bending. Device 910 may be device 100 described above with respect to Figs. 1A, 1B, 1C, 1D and 1E and Figs. 2A, 2B, 2C, 2D, 2E and 2F, device 200 as described above with respect to Figs. 3A, 3B, 3C and 3D, and/or device 300 as described above with respect to Fig. 4.

Elongated element 90 and/or elongated element 90 supported within device 910 may be accommodated within envelope 911, thus providing an elongated assembly 914. Envelope 911 may, for example, be envelope 330, envelope 340 or envelope 350 described above with respect to Figs. 4A, 4B and 4C, respectively.

Heating device 920 may receive elongated assembly 914 and elevate a temperature of elongated element 90 disposed therein up to the predetermined temperature pressure (e.g., 380°C). Heating device 920 may be heating device 400 described above with respect to Fig. 5.

Bending device 930 may receive elongated assembly 914 and bend elongated assembly 914, and particularly elongated element 90 disposed therein, based on the predetermined bending profile. Bending device 930 may be any of bending devices described above with respect to Figs. 6A, 6B and 6C, Figs, 7A, 7B, 7C, 7D and 7E, Figs. 8A, 8B, 8C, 8D and 8E, and Figs. 9A and 9B.

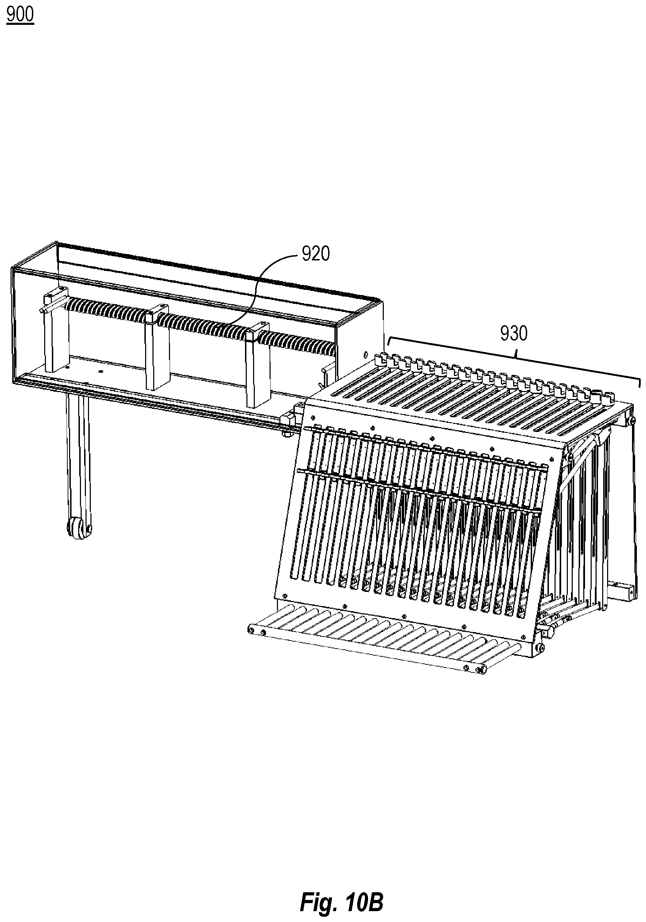

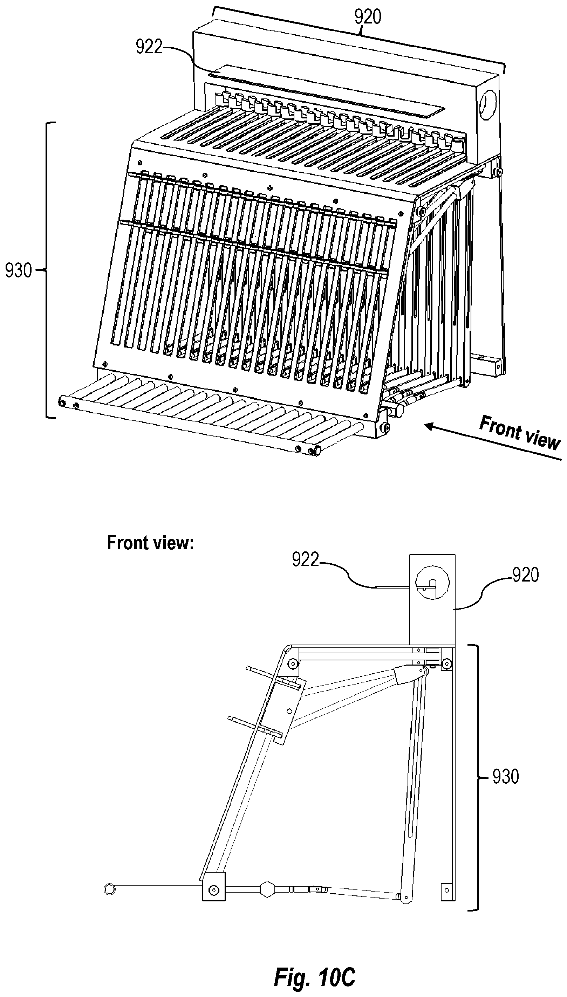

Reference is now made to **Figs. 10B** and **10C**, which are schematic illustrations of various configurations of coupling of a heating device 920 and a bending device 930 useful for understanding the invention.

Heating device 920 may be positioned adjacent to bending device 930 at a height that enables pulling elongated assembly 914 from heating device 920 into bending device 930 (e.g., as shown in Fig. 10B).

Heating device 920 may be positioned above bending device 930 (e.g., as shown in Fig. 10C). Heating device 920 may include a supporting plate 922. Supporting plate 922 may support elongated assembly 914 when accommodated within heating device 920. Upon heating of elongated element 90 to the predetermined temperature value, supporting plate 922 may be removed, thereby letting elongated assembly to fall into bending device 930.

It is noted that, although Figs. 10B and 10C show bending device 930 similar to that described above with respect to Figs. 7A-7E, any bending device of those described above with respect Figs. 6A, 6B and 6C, Figs, 7A, 7B, 7C, 7D and 7E, Figs. 8A, 8B, 8C, 8D and 8E, and Figs. 9A and 9B may be used.

Reference is now made to Fig. 11, which is a schematic illustration of a device 1000 for determining a desired bending profile of an elongated element 90 useful for understanding the invention.

Device 1000 may include a housing 1100 and at least one sensor 1200 embedded in housing 1100 (e.g., as shown in Fig. 11). Sensor(s) 1200 may be capable of determining locations of target zones 70. Sensor(s) 1200 may include at least one of: GPS, accelerometer, gyro, electromagnetic field sensor, hall sensor, LIDAR, Radar, or any combination thereof.

When elongated element 90 is an implant, target zones 70 may, for example, be points of connection of the implant (e.g., elongated element 90) to anatomical zone 70. For example, Fig. 11 shows spinal segments as an example of anatomical zone 70 and pedicle screws as an example of target zones 70. In this example, pedicle screws (e.g., target zones 70) are arranged to receive and support a rod (e.g., elongated element 90) that has to be bent to accurately mimic the geometry of anatomical zone 70.

Housing 1100 may be a hand-held device and may be manually operated by the user (e.g., by the surgeon).

Device 1000 may include a motoric unit 1300 mechanically coupled with housing 1100. Motoric unit 1300 may be arranged to operate (e.g., move) housing 1100.

In order to obtain data concerning the locations of target zones 70, housing 1100 (e.g., operated by the user or by motoric unit 1300) may subsequently touch each of target zones 70, and sensor(s) 1200 may generate the data concerning the locations of target zones 70.

Device 1000 may include a controller 1400. Controller 1400 may be in communication (e.g., wired or wireless) with sensor(s) 1200 and may be configured to receive from sensor(s) 1200 the data concerning the locations of target zones 70.

Controller 1400 may be configured to determine, based on the data concerning the locations of target zones 70, the desired bending profile of elongated element 90.

Controller 1400 may be in communication with a controller of a bending device (e.g., such as controller 740 of bending device 700 as described above with respect to Fig. 8A). Controller 1400 may be configured to transmit the desired bending profile to the controller of the bending device, which may be configured to operate the bending units of the bending device (e.g., such as bending units 710 as described above with respect to Fig. 8A-8E) to bend elongated element 90 based on the desired bending profile thereof.

The data concerning the locations of target zones 70 may be transmitted to the controller of the bending device, and the controller of the bending device may be further configured to determine the desired bending profile based on the data thereof.

Controller 1400 may generate an output including instructions concerning manual operation of the bending units of the bending device (e.g., such as bending device 600 described above with respect to Figs. 7A-7E).

Device 1000 may be part of a system for bending elongated element 90 (e.g., such as system 900 described above with respect to Figs. 10A-10C).

Alternatively or complementarily, the desired bending profile of elongated element 90 may be determined by any imaging system such as, for example, computed tomography (CT), magnetic resonance imaging (MRI), X-Ray, navigation system, etc., prior to and/or during the operational procedure.

Alternatively or complementarily, a deformable template may be used to determine the desired bending profile of elongated element 90. The deformable template may, for example, have shape and dimensions that may be similar to shape and dimensions of elongated element 90. The template may be manually deformed by the user to pass through target zones 70 and fit anatomical zone 70. The deformed template may be then positioned in the bending device the bending units of which may then be operated according to the deformed template.

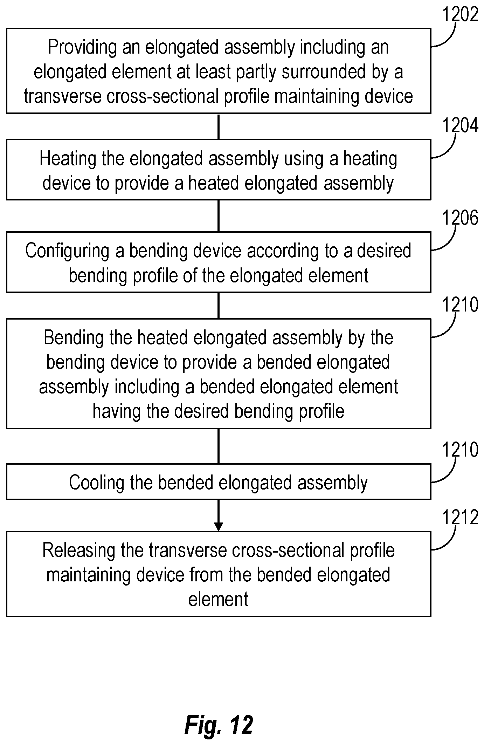

Reference is now made to Fig. 12, which is a flowchart of a method of bending of an elongated assembly, according to some embodiments of the invention.

The method of bending of an elongated assembly includes providing an elongated assembly, including an elongated orthopedic implant comprising a thermoplastic polymer, the elongated orthopedic implant comprising a bending region in which the elongated orthopedic implant is to be bent, the bending region having a length; and a transverse cross-sectional profile maintaining device surrounding completely and tightly supporting the elongated orthopedic implant along the entire length of the bending region of the elongated orthopedic implant for maintaining a shape and preventing distortion of a transverse cross-sectional profile of the elongated orthopedic implant in the bending region upon heating (stage 1202).

The elongated assembly may be similar to elongated assembly 80 described hereinabove. The elongated element may be similar to elongated element 90 described hereinabove. The transverse cross-sectional profile maintaining device may be similar to, for example, device 100 described above with respect to Figs. 1A-1E, Figs. 2A-2F and device 200 described above with respect to Figs. 3A-3B.

The method includes heating the elongated assembly using a heating device to provide a heated elongated assembly (stage 1204). The heating device may be similar to heating device 400 described above with respect to Fig. 5.

The method includes configuring a bending device according to a desired bending profile of the elongated orthopedic implant (stage 1206).

The bending device may be similar to, for example, bending device 500 described above with respect to Figs. 6A-6C, bending device 600 described above with respect to Figs. 7A-7E, bending device 700 described above with respect to Figs. 8A-8E, and bending device 800 described above with respect to Figs. 9A-9B.

The desired bending profile may be predetermined using, for example, device 1000 described above with respect to Fig. 11, an imaging system (e.g., CT, MRI, X-Ray, etc.) or a deformable template.

The method includes bending the heated elongated assembly by the bending device to provide a bended elongated assembly including a bended elongated orthopedic implant having the desired bending profile while maintaining the shape and preventing the distortion of the transverse cross-sectional profile of the elongated orthopedic implant in the bending region by the transverse cross-sectional profile maintaining device (stage 1208). For example, as described above with respect to any one of Figs. 6A-6C, Figs. 7A-7E, Figs. 8A-8E and Figs. 9A-9B.

The method includes cooling the bended elongated assembly (stage 1210). The cooling may be, for example, active. In another example, the cooling may be passive.

The method includes releasing the transverse cross-sectional profile maintaining device from bended elongated orthopedic implant (stage 1212). For example, as described above with respect to any one of Figs. 1A-1E, Figs. 2A-2F and Figs. 3A-3B.

In some embodiments, the elongated assembly, the heating device and the bending device may be sterile and sterilizable. For example, the heating device may include a heating envelope (e.g., such as heating envelope 430 described above with respect to Fig. 5) and bending device may be such as bending device 600 described above with respect to Figs. 7A-7E or bending device 700 described above with respect to Figs. 8B-8E.

In some embodiments, at least one of the heating device and the bending device may be not sterile. In this case, the elongated assembly may include an envelope (e.g., such as envelope 330, 340, 350 described above with respect to Figs. 4A, 4B and 4C, respectively) that at least partly envelopes sterile elongated orthopedic implant at least partly surrounded by sterile transverse cross-sectional profile maintaining device.

Advantageously, the disclosed devices, assemblies, kits, systems and methods for shaping of elongated elements containing thermoplastic polymers may enable shaping of the elongated elements containing thermoplastic elements while preventing distortion of the shape of the cross-sectional profile in the shaping region. Furthermore, the disclosed devices and systems may be located in the operation room and may enable shaping of the elongated elements thereof in the operation room during the operational procedure.

## Claims

1. A method of bending of an elongated assembly (202, 301), the method comprising:
providing an elongated assembly comprising:
an elongated orthopedic implant (100, 200, 310) comprising a thermoplastic polymer, the elongated orthopedic implant comprising a bending region in which the elongated orthopedic implant is to be bent, the bending region having a length; and
a transverse cross-sectional profile maintaining device surrounding completely and tightly supporting the elongated orthopedic implant along the entire length of the bending region of the elongated orthopedic implant for maintaining a shape and preventing distortion of a transverse cross-sectional profile of the elongated orthopedic implant in the bending region upon heating;
heating the elongated assembly using a heating device (400) to provide a heated elongated assembly;
configuring a bending device (500, 600, 700, 800, 930) according to a desired bending profile of the elongated orthopedic implant;
bending the heated elongated assembly by the bending device to provide a bended elongated assembly comprising a bended elongated orthopedic implant having the desired bending profile while maintaining the shape and preventing the distortion of the transverse cross-sectional profile of the elongated orthopedic implant in the bending region by the transverse cross-sectional profile maintaining device;
cooling the bended elongated assembly; and
releasing the device for maintaining the shape of the transverse cross-sectional profile from the elongated orthopedic implant.

2. The method of claim 1, wherein at least one of the heating device (400) and the bending device (500, 600, 700, 800, 930) is not sterile, and wherein the elongated assembly comprises an envelope (330, 340, 350) that at least partly envelopes sterile elongated orthopedic implant at least partly surrounded by sterile transverse cross-sectional profile maintaining device.

3. The method of claim 2, wherein the envelope comprises one or more caps each adapted to close one end of the envelope.

4. The method of claim 3, wherein at least one cap of the one or more caps is screwable to the respective end of the envelope.

## Patentansprüche

1. Verfahren zum Biegen einer länglichen Baugruppe (202, 301), wobei das Verfahren Folgendes umfasst:
Bereitstellen einer länglichen Baugruppe, Folgendes umfassend:
ein längliches orthopädisches Implantat (100, 200, 310), das ein thermoplastisches Polymer umfasst, wobei das längliche orthopädische Implantat einen Biegebereich umfasst, in dem das längliche orthopädische Implantat gebogen werden soll, wobei der Biegebereich eine Länge aufweist; und
eine Vorrichtung zum Beibehalten eines transversalen Querschnittsprofils, die das längliche orthopädische Implantat entlang der gesamten Länge des Biegebereichs des länglichen orthopädischen Implantats vollständig umschließt und fest stützt, um eine Form beizubehalten und eine Verformung eines transversalen Querschnittsprofils des länglichen orthopädischen Implantats in dem Biegebereich beim Erhitzen zu verhindern;
Erhitzen der länglichen Baugruppe unter Verwendung einer Heizvorrichtung (400), um eine erhitzte längliche Baugruppe bereitzustellen;
Konfigurieren einer Biegevorrichtung (500, 600, 700, 800, 930) gemäß einem gewünschten Biegeprofil des länglichen orthopädischen Implantats;
Biegen der erhitzten länglichen Baugruppe durch die Biegevorrichtung, um eine gebogene längliche Baugruppe bereitzustellen, die ein gebogenes längliches orthopädisches Implantat mit dem gewünschten Biegeprofil umfasst, während die Form beibehalten wird und die Verformung des transversalen Querschnittsprofils des länglichen orthopädischen Implantats in dem Biegebereich durch die Vorrichtung zum Beibehalten des transversalen Querschnittsprofils verhindert wird;
Abkühlen der gebogenen länglichen Baugruppe; und
Lösen der Vorrichtung zum Beibehalten des transversalen Querschnittsprofils von dem länglichen orthopädischen Implantat.

2. Verfahren nach Anspruch 1, wobei mindestens eine von der Heizvorrichtung (400) und der Biegevorrichtung (500, 600, 700, 800, 930) nicht steril ist und wobei die längliche Baugruppe eine Hülle (330, 340, 350) umfasst, die mindestens teilweise ein steriles längliches orthopädisches Implantat umhüllt, das mindestens teilweise durch eine sterile Vorrichtung zum Beibehalten des transversalen Querschnittsprofils umschlossen ist.

3. Verfahren nach Anspruch 2, wobei die Hülle eine oder mehrere Kappen umfasst, die jeweils angepasst sind, ein Ende der Hülle zu verschließen.

4. Verfahren nach Anspruch 3, wobei mindestens eine Kappe der einen oder mehreren Kappen auf das jeweilige Ende der Hülle schraubbar ist.

## Revendications

1. Procédé de cintrage d'un ensemble allongé (202, 301), le procédé comprenant :
la fourniture d'un ensemble allongé comprenant :
un implant orthopédique allongé (100, 200, 310) comprenant un polymère thermoplastique, l'implant orthopédique allongé comprenant une région de cintrage dans laquelle l'implant orthopédique allongé doit être cintré, la région de cintrage ayant une longueur ; et
un dispositif de maintien du profil de section transversale entourant complètement et soutenant étroitement l'implant orthopédique allongé sur toute la longueur de la région de cintrage de l'implant orthopédique allongé pour maintenir une forme et empêcher une déformation d'un profil de section transversale de l'implant orthopédique allongé dans la région de cintrage lors du chauffage ;
le chauffage de l'ensemble allongé à l'aide d'un dispositif de chauffage (400) pour obtenir un ensemble allongé chauffé ;
la configuration d'un dispositif de cintrage (500, 600, 700, 800, 930) selon un profil de cintrage souhaité de l'implant orthopédique allongé ;
le cintrage de l'ensemble allongé chauffé par le dispositif de cintrage pour obtenir un ensemble allongé cintré comprenant un implant orthopédique allongé cintré ayant le profil de cintrage souhaité, tout en maintenant la forme et en empêchant la déformation du profil de section transversale de l'implant orthopédique allongé dans la région de cintrage au moyen du dispositif de maintien du profil de section transversale ;
le refroidissement de l'ensemble allongé cintré ; et
le retrait du dispositif de maintien de la forme du profil de section transversale de l'implant orthopédique allongé.

2. Procédé selon la revendication 1, dans lequel au moins l'un du dispositif de chauffage (400) et du dispositif de cintrage (500, 600, 700, 800, 930) n'est pas stérile, et dans lequel l'ensemble allongé comprend une enveloppe (330, 340, 350) qui enveloppe au moins partiellement un implant orthopédique allongé stérile au moins partiellement entouré d'un dispositif de maintien du profil de section transversale stérile.

3. Procédé selon la revendication 2, dans lequel l'enveloppe comprend un ou plusieurs capuchons chacun adapté pour fermer une extrémité de l'enveloppe.

4. Procédé selon la revendication 3, dans lequel au moins un capuchon parmi les un ou plusieurs capuchons peut être vissé sur l'extrémité respective de l'enveloppe.
